# EUROPEAN PATENT APPLICATION

(11) **EP 4 560 009 A1**
(43) Date of publication of application: **28.05.2025**
(21) Application number: 23212248.1
(22) Date of filing: 27.11.2023
(51) Int. Cl.: C12N 5/071, A61K 9/00, A61K 35/407, A61P 1/16, G01N 33/50

(54) **METHOD FOR THE PREPARATION OF ISOLATED LIVER-DERIVED CELLS**

(71) Applicant: Cellaïon SA, 1435 Mont-Saint-Guibert (BE)
(72) Inventor: SOKAL, Etienne, 1435 Mont Saint Guibert (BE); NAJIMI, Mustapha, 1435 Mont Saint Guibert (BE)
(74) Representative: Icosa

(57) **Abstract**

The current invention relates to a method for obtaining a population of isolated liver-derived cells suitable for therapeutic use, wherein said liver-derived cells are obtained from isolated adult livers with metabolic defects or a part thereof. The invention also relates to a suspension of primary liver cells obtained from isolated adult livers with metabolic defects or a part thereof, preferably wherein said primary liver cells have a metabolic defect; and to a liver cell population obtained from isolated adult livers with metabolic defects or a part thereof. The invention further relates to uses of said suspension of primary liver cells and/or said liver cell population.

## Description

### FIELD OF THE INVENTION

The present invention relates to a method for obtaining a population of isolated liver-derived cells suitable for therapeutic use, wherein said liver-derived cells are obtained from isolated adult livers with metabolic defects or a part thereof. The invention also relates to a suspension of primary liver cells and to a liver cell population both obtained from isolated adult livers with metabolic defects or a part thereof. The invention further relates to uses of said suspension of primary liver cells and/or said liver cell population.

### BACKGROUND

The use of stem or progenitor cells, in particular liver progenitor cells has been identified in the literature using liver tissues from different organisms, as well as in fetal or adult liver tissues (Schmelzer E et al., 2007; Sahin MB et al., 2008; Azuma H et al., 2003; Herrera MB et al., 2006; Najimi Met al., 2007; Darwiche Hand Petersen BE, 2010; Shiojiri N and Nitou M, 2012; Tanaka Mand Miyajima A, 2012, El-Kehdy et al, 2016). Such cells are believed to potentially provide, following the exposure to hepatogenic stimuli in vitro and/ or after in vivo administration, cells with morphological and functional features typically associated to hepatic differentiation such as phase I/II enzymatic activities.

WO2016/030525 and WO2017/149059 disclose specific cell culture conditions allowing the obtention of human adult liver-derived progenitor cells (HALPC) with specific expression profile and improved biological features. For example, WO2016/030525 describes the use of basal media formulations such as William's medium E, Dulbecco's Modified Eagle's Medium (DMEM) preferably complemented with mammalian plasma or sera in particular fetal bovine (calf) serum (i.e., FCS or FBS) in a process of culturing said cells. It is also described in WO2016/030525, that besides providing nutrients and/or growth promoters, the liquid culture media with addition of bovine, human or other animal serum may also promote the growth/adherence or the elimination/detachment of specific cell types. WO2020/221843 discloses a process for the manufacturing of a population of human allogeneic liver-derived progenitor cells (HALPC) with the use of a xeno- and serum-free culture medium comprising purified native or recombinant human serum albumin. WO2020/221842 discloses a process for the manufacturing of a population of human allogeneic liver-derived progenitor cells (HALPC), comprising the use of microcarriers and a bioreactor. The primary liver cell suspension (LCS) used as starting material for the manufacturing of HALPC is obtained from human healthy livers (without abnormalities), generally human livers (or parts thereof) from a deceased donor that is deemed not eligible for transplant. In order to overcome the shortage of eligible livers for HALPC preparation, we need to find new sources of human liver tissues.

The liver is a key organ in the regulation of body homeostasis and is the site of many vital metabolic pathways. Impairment of only one protein within a complex metabolic pathway could be highly deleterious. The large presence of important liver enzymes substantially increases the risk of occurrence of diverse liver diseases. Inborn errors of liver metabolism comprise various genetic diseases that alter the activity of the liver. Altogether, 200 different inborn errors of liver metabolism exist. The symptoms caused and related to such metabolic defects may appear as early as in the neonatal period. Inborn errors of liver metabolism, which incidence is estimated to be 1: 1000 live birth, result from single gene defects and significantly disturb metabolic processes including the accumulation of a toxic substrate, lack of a product or failure of energy production. The impact of such genetic alterations is dependent on the pathway affected. It may structurally damage the liver and some peripheral organs in some cases (Alpha-1-antitrypsin deficiency) or it may only affect the concerned pathway in the liver which remains structurally healthy while peripheral organs may be secondarily affected (urea cycle disorders). Even though each metabolic disease displays specific characteristics, clinical signs generally include lethargy, decreased feeding, vomiting, acidosis, and seizures. Such clinical features may be acutely presented at neonatal and/or chronically exhibited in childhood, adolescence or even adulthood. Therefore, early diagnosis and appropriate treatments are crucial to limit the exacerbation of the impaired functions. Indeed, interventions via specific diets will aim at eliminating the toxic metabolite (by scavengers) or the substrate for which the enzyme is lacking, as well as the supplementation of the missing downstream product. Such conservative treatments failed sometimes despite an optimal management which leads to serious health problems for the patients and the necessity for an orthotopic liver transplantation (OLT). Indeed, inborn errors of liver metabolism are the second indication (~10%) for pediatric OLT which has been shown to cure some of those diseases or only improve the clinical outcomes for others. Moreover, OLT is highly invasive, irreversible, limited by shortage of donor grafts and demands state-of-art surgery.

To address the shortage of donor liver organs for OLT, many strategies have been developed aiming to increase organ availability for Liver Transplantation (LT). Domino liver transplantation (DLT) uses explanted livers from transplant recipients as graft for other patients (Buijk *et al.*)*.* This re-use of explanted livers from transplant patients is possible in a limited number of diseases and the development of the donor's disease in the domino recipient does not happen or takes longer than post-transplant life expectancy. Otherwise, the post LT graft function is normal. Typical examples are livers from metabolic disease patients re-used in elderly recipients with cancer as underlying indication for LT. Examples of the metabolic diseases forming an indication for domino LT are: familial amyloid neuropathy, fibrinogen A2 chain amyloidosis, maple syrup urine disease, familial hypercholesterolemia, and neurogenic intestinal pseudo-obstruction. The domino recipient (or patient) can receive a liver from a deceased or a living donor. Nevertheless, the number of possible DLT procedures will remain a very low percentage of the overall LT numbers. Therefore, the usage of this option will not have a statistical effect on the waitlist mortality in a country or region.

Liver cell transplantation (LCT) may exert only short-to-medium term efficacy due to the quality of hepatocyte preparations. Moreover, major challenges for the wide clinical application of hepatocyte therapy include availability, metabolic integrity, sufficient amount of cells for transplantation, and poor resistance of hepatocytes to in vitro culture and cryopreservation conditions. The domino concept has been applied for hepatocyte transplantations (Monteiro da Fonseca Cardoso *et al.*)*.* For the first time in LCT, Stephenne *et al.* applied the domino procedure by which cells from a metabolic patient, type 1b glycogen storage disease in this particular case, are used to cure a different metabolic condition. The group of Gramignoli *et al.* compared hepatocytes isolated from explants of normal livers and livers presenting various types of inherited metabolic diseases and/or conditions, and demonstrated, by using liver function assays, such as drug and ammonia metabolism and conjugation, that the cells from most metabolic disease cases were able to perform these functions as well as, or better than, cells from normal donors.

Accordingly, a need arises for novel methods for the preparation of isolated liver-derived cells suitable for therapeutic use, in particular of HALPC, in order to overcome shortage of eligible healthy livers.

The present invention aims to resolve at least some of the problems and disadvantages mentioned above.

### SUMMARY OF THE INVENTION

The present invention and embodiments thereof serve to provide a solution to one or more of above-mentioned disadvantages.

To this end, the present invention may be described by the following embodiments:
1. A method for obtaining a population of isolated liver-derived cells suitable for therapeutic use, wherein said liver-derived cells are obtained from isolated adult livers with metabolic defects or a part thereof.
2. The method according to embodiment 1, wherein said adult livers with metabolic defects or parts thereof are adult livers or parts thereof displaying impaired metabolic functions linked to genetic mutations and/or enzymatic deficiencies.
3. The method according to embodiment 2, wherein said genetic mutations or enzymatic deficiencies result in a metabolic liver disease or disorder, wherein said liver disease or disorder is preferably chosen from the group of familial amyloidotic polyneuropathy (FAP), maple syrup urine disease (MSUD), acute intermittent porphyria, hereditary fibrinogen A o-chain amyloidosis, propionic acidemia, hyperhomocysteinemia, methylmalonic acidemia, familial hypercholesterolemia, primary hyperoxaluria, hemophilia A, urea cycle disorders, and Crigler Najjar syndrome.
4. The method according to any one of the previous embodiments, wherein said liver-derived cells are obtained from one isolated adult liver with one metabolic defect, or a part thereof.
5. The method according to any of the previous embodiments, wherein said adult liver or part thereof is obtained from a donor, preferably a human donor, wherein said liver is isolated from said donor, or wherein said part of said liver is obtained from said donor via a biopsy.
6. The method according to embodiment 5, comprising a step of screening said donor for the presence of metabolic liver diseases or disorders.
7. The method according to any one of the previous embodiments, comprising a step of screening said adult liver or part thereof for the presence of metabolic defects.
8. The method according to any one of the previous embodiments, wherein the liver with metabolic defects or a part thereof shows an unaltered structure compared to such liver or part thereof without said metabolic defects.
9. The method according to any one of the previous embodiments, wherein said liver-derived cells are chosen from liver progenitor cells such as adult liver progenitor cells, hepatocyte progenitors, cholangiocyte progenitors, stellate cell progenitors, liver endothelial cell progenitors, and/or Kupffer cell progenitors.
10. The method according to any one of the previous embodiments, wherein said liver-derived cells are adult liver progenitor cells.
11. The method of embodiment 10, further establishing that said adult liver progenitor cells express at least one mesenchymal marker selected from CD90, CD44, CD73, CD13, CD140b, CD29, vimentin and alpha-smooth muscle actin (ASMA), and optionally expressing at least one hepatic marker and/or exhibiting at least one liver-specific activity.
12. The method according to any one of the previous embodiments, wherein said liver-derived cells are for allogeneic use.
13. The method according to any of the previous embodiments, comprising:
   a) disassociating adult liver with metabolic defects or a part thereof to form a population of primary liver cells having said metabolic defects;
   b) generating a preparation of the population of primary liver cells of step a), thereby obtaining a suspension of primary liver cells;
   c) culturing and expanding said suspension of primary liver cells of step b); and
   d) harvesting said expanded cell population of step c).
14. The method according to embodiment 13, wherein the primary liver cells of step a) are of human origin.
15. A suspension of primary liver cells obtained from isolated adult livers with metabolic defects or a part thereof.
16. The suspension of primary liver cells according to embodiment 15, obtained from one isolated adult liver with one metabolic defect, or a part thereof.
17. The suspension of primary liver cells according to embodiment 15 or 16, wherein said primary liver cells have a metabolic defect.
18. The suspension of primary liver cells according to any of embodiment 15 to 17, wherein the suspension is obtained via steps a) and b) of the method according to embodiment 13 or 14.
19. The suspension of primary liver cells according to any of embodiments 16 to 18, wherein the suspension is cryopreserved.
20. The use of the suspension of primary liver cells according to any of embodiments 16 to 19 in the preparation of a population of isolated liver-derived cells for therapeutic use.
21. A liver cell population obtained from isolated adult livers with metabolic defects or a part thereof, comprising liver-derived cells obtained by a method of any one of the embodiments 1 to 14, or obtained by culturing and expanding the suspension of primary liver cells according to any one of embodiments 15 to 19 and subsequently harvesting said expanded cell population.
22. The liver cell population according to embodiment 21, wherein said liver-derived cells have a metabolic defect.
23. The liver cell population according to any one of embodiments 21 to 22 or the suspension of primary liver cells according to any one of embodiments 15 to 19, wherein the cells have metabolic defects, said metabolic defects are linked to one or more genetic mutations and/or enzymatic deficiencies resulting in a metabolic liver disease or disorder, said liver disease or disorder is preferably chosen from the group of familial amyloidotic polyneuropathy (FAP), maple syrup urine disease (MSUD), acute intermittent porphyria, hereditary fibrinogen A o-chain amyloidosis, propionic acidemia, hyperhomocysteinemia, methylmalonic acidemia, familial hypercholesterolemia, primary hyperoxaluria, hemophilia A, urea cycle disorders, and Crigler Najjar syndrome.
24. A pharmaceutical composition comprising a therapeutically effective amount of the liver cell population according to any one of the embodiments 21 to 23.
25. The liver cell population of any one of embodiments 21 to 23, or the pharmaceutical composition of embodiment 24 for use as a medicament.
26. The liver cell population of any one of embodiments 21 to 23, or the pharmaceutical composition of embodiment 24 for use in treating a liver disease or condition in a subject.
27. The liver cell population or pharmaceutical composition for use of embodiment 26, wherein the liver disease or condition is selected from the group of phenylketonuria and other aminoacidopathies, hemophilia and other clotting factor deficiencies, familial hypercholesterolemia and other lipid metabolism disorders, urea cycle disorders, glycogenosis, galactosemia, fructosemia, tyrosinemia, protein and carbohydrate metabolism deficiencies, organic aciduria, mitochondrial diseases, peroxysomal and lysosomal disorders, protein synthesis abnormalities, defects of liver cell transporters, defects of glycosylation, cirrhosis, inborn errors of metabolism, acute liver failure, acute liver infections, acute chemical toxicity, chronic liver failure, fulminant liver failure, cholangitis, biliary cirrhosis, Alagille syndrome, alpha-1-antitrypsin deficiency, autoimmune hepatitis, biliary atresia, cancer of the liver including hepatocellular carcinoma (HCC), cystic disease of the liver, gallstones, Gilbert's syndrome, hemochromatosis, hepatitis A, hepatitis B, hepatitis C, and other hepatitis viral infections, porphyria, primary sclerosing cholangitis, Reye's syndrome, sarcoidosis, type 1 glycogen storage disease, Wilson's disease, an inherited Blood Coagulation Disorder, progressive familial intrahepatic cholestasis type 1 / 2 / 3, defect of liver cell transporters, fatty liver, fibrotic liver disease, Acute-on- chronic liver failure (ACLF) and non-alcoholic fatty liver disease (NAFLD), nonalcoholic steatohepatitis (NASH), or liver degenerative disease.
28. The liver cell population or pharmaceutical composition for use of embodiments 26 to 27, wherein said cell population or pharmaceutical composition is administered to said subject, said subject is suffering from or at risk of developing a liver disease or disorder, and wherein the liver disease or disorder to be treated of said subject is not caused by or linked to a metabolic defect similar to that present in said administered cells.
29. The liver cell population of any of the embodiments 21 to 23, or the pharmaceutical composition of embodiment 24, for use in modulating or influencing impaired vascular permeability in (cells of) a subject; and/or for use in the treatment of diseases and/or conditions caused by increased vascular permeability, preferably wherein said diseases and/or conditions caused by increased vascular permeability are chosen from the group of heart, pulmonary and ischemic diseases, diabetes and ocular diseases, cancer (solid tumors), Clarkson's disease and sepsis in a subject, preferably triggered by an infection and/or is sepsis or a sepsis-induced disease such as sepsis-induced myocardial edema, sepsis-induced acute kidney injury, lung sepsis, or Clarkson's disease.
30. The liver cell population of any of the embodiments 21 to 23, or the pharmaceutical composition of embodiment 24, for use in the treatment and/or prevention of one of more diseases related to cellular senescence selected from the group comprising or consisting of hepatic fibrosis, pre-cirrhotic conditions, cirrhosis including liver and biliary cirrhosis, biliary atresia, Alagille syndrome, progressive familial intrahepatic cholestasis, primary biliary cholangitis, primary sclerosing cholangitis, chronic hepatitis, chronic hepatitis B virus (HBV) infection, chronic hepatitis C virus (HCV) infection, cholestasis, osteoporosis, osteoarthritis, atherosclerosis, cardiac hypertrophy, cardiac fibrosis, cardiomyopathy, thrombosis, cataracts, glaucoma, macular degeneration, chronic obstructive pulmonary disease (COPD), pulmonary fibrosis, renal dysfunction, pancreatic fibrosis, type 2 diabetes, Alzheimer disease, Huntington's disease, Parkinson's disease, dementia, lipodystrophy, sarcopenia, age-related cachexia, skin aging, hepatocellular carcinoma, liver cancer, lobular carcinoma, bile duct cancer, melanoma, lung cancer, and islet cell tumor.
31. The liver cell population or pharmaceutical composition for use of embodiments 25 to 30, wherein said liver cell population or pharmaceutical composition is administered to a subject, and wherein the metabolic defect of said administered cells is absent in said subject.
32. The pharmaceutical composition for use according to any one of embodiments 25 to 31 wherein said pharmaceutical composition is a suspension of isolated liver-derived cells that is suitable for intrahepatic, intrasplenic, intraportal, or intravenous administration.
33. A method of obtaining a population of isolated liver-derived cells, suited for use in a patient suffering from a liver disease or condition and/or a disease or condition caused by increased vascular permeability and/or a disease related to cellular senescence, comprising isolating liver-derived cells from a donor liver or part thereof with metabolic defects, preferably wherein said metabolic defects from said donor liver are absent in said subject.
34. A method of obtaining a population of isolated liver-derived cells, suited for use as a personalized medicine in a patient suffering from a liver disease or condition and/or a disease or condition caused by increased vascular permeability and/or a disease related to cellular senescence, comprising isolating liver-derived cells from a donor liver or part thereof with metabolic defects, wherein said metabolic defects of said donor liver are absent in said patient, and administering said cells to said patient.
35. A method of obtaining a population of isolated liver-derived cells, suited to be administered to a subject, said method comprising screening the subject for the presence of metabolic liver defects, and isolating liver-derived cells from a donor liver or part thereof having metabolic defects.
36. The method according to any one of the previous embodiments 33 to 35, wherein said liver-derived cells are chosen from the list of liver progenitor cells such as adult liver progenitor cells, hepatocyte progenitors, cholangiocyte progenitors, stellate cell progenitors, liver endothelial cell progenitors, and/or Kupffer cell progenitors.

The methods presented herein, along with the resulting suspension of primary liver cells and liver cell population derived from adult livers with metabolic defects, offer a transformative solution to the shortage of donor liver organs, alleviating the challenges associated with wait-list mortality in diverse countries and regions. This approach leverages adult livers typically dismissed as waste material, unlocking a valuable resource for therapeutic use. By harnessing liver-derived cells from livers with metabolic defects, the invention broadens the pool of available organs. This advancement holds significant promise in treating a wide range of diseases, contributing to improved patient outcomes and reducing reliance on traditional organ sources.

### DETAILED DESCRIPTION OF THE INVENTION

The present invention concerns a method for obtaining a population of isolated liver-derived cells suitable for therapeutic use, wherein said liver-derived cells are obtained from isolated adult livers with metabolic defects or a part thereof. The invention also relates to a suspension of primary liver cells, to a liver cell population, and uses thereof.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention. The terms or definitions used herein are provided solely to aid in the understanding of the invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, elements, members, steps, known in the art or disclosed therein.
Furthermore, the terms "first", "second", "third" and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.,* any ≥3, ≥4, ≥5, ≥6 or ≥7 etc. of said members, and up to all said members.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the present invention relates to a method for obtaining a population of isolated liver-derived cells suitable for therapeutic use, wherein said liver-derived cells are obtained from isolated adult livers with metabolic defects or a part thereof.

In a preferred embodiment, said liver-derived cells are obtained from one isolated adult liver or a part thereof. In another or further preferred embodiment, said isolated adult liver(s) have one metabolic defect.

In a most preferred embodiment, said liver-derived cells are obtained from one isolated adult liver with one metabolic defect or a part thereof.

In an embodiment, said adult livers with metabolic defects or parts thereof are adult livers or parts thereof displaying impaired metabolic functions linked to genetic mutations and/or enzymatic deficiencies.

The term "liver" refers to liver organ. The term "part of liver" generally refers to a tissue sample derived from any part of the liver organ, without any limitation as to the quantity of the said part or the region of the liver organ where it originates. Preferably, all cell types present in the liver organ may also be represented in the said part of liver. Quantity of the part of liver may at least in part follow from practical considerations to the need to obtain enough primary liver cells for reasonably practicing the method of the invention. Hence, a part of liver may represent a percentage of the liver organ (e.g. at least 1%, 10%, 20%, 50%, 70%, 90% or more, typically w/w). In other non-limiting examples, a part of liver may be defined by weight (e.g. at least 1g, 10g, 100g, 250g, 500g, or more). For example, a part of liver may be a liver lobe, e.g., the right lobe or left lobe, or any segment or tissue sample comprising a enough number of cells that is resected during split liver operation or in a liver biopsy.

The term "adult liver" refers to liver of subjects that are post-natal, i.e. any time after birth, preferably full term, and may be, e.g., at least 1 day, 1 week, 1 month or more than 1 month of age after birth, or at least 1, 5, 10 years or more. Hence, an "adult liver", or mature liver, may be found in human subjects who would otherwise be described in the conventional terms of "infant", "child", "adolescent", or "adult". The liver or part thereof is obtained from a "subject" or "donor", interchangeably referring to a vertebrate animal, preferably a mammal, more preferably a human. In another embodiment, the adult liver or part thereof may be from a non-human animal subject, preferably a non-human mammal subject (e.g. a rodent or pig).

In an embodiment, said adult liver or part thereof is obtained from a donor, preferably a human donor. Said liver is isolated from said donor, or said part of said adult liver is obtained from said donor via a biopsy.

A donor may be living or dead, as determined by clinically accepted criteria, such as the "heart-lung" criteria (involving an irreversible cessation of circulatory and respiratory functions) or the "brain death" criteria (involving an irreversible cessation of all functions of the entire brain, including the brainstem). Harvesting may involve known procedures such as biopsy, resection or excision. Harvesting of liver tissue from a living human donor may need to be compatible with sustenance of further life of the donor. The liver or part thereof may be obtained from a donor, esp. human donor, who has sustained circulation, e.g., a beating heart, and sustained respiratory functions, e.g., breathing lungs or artificial ventilation. Only a part of liver may typically be removed from a living human donor (e.g., by biopsy or resection), such that an adequate level of normal liver functions is maintained in the donor, as required by legal and ethical norms.

Subject to ethical and legal norms, the donor may need to be or need not be brain dead. For instance, the removal of an entire liver or portion thereof, which would not be compatible with further survival of a human donor, may be allowed in brain dead human beings. Alternatively, the removal of the entire liver is compatible with the further survival of said human donor if subsequently transplanted with another liver.

This may be exemplified in the case of a donor with a metabolic defect, where the liver is removed and replaced with another liver, preferably one without such metabolic defect. Harvesting of liver or part thereof from such donors is advantageous, since the tissue does not suffer substantial anoxia (lack of oxygenation), which usually results from ischemia (cessation of circulation). At the time of harvesting the tissue, the donor may have ceased circulation and/or respiratory functions, with no artificial ventilation. While liver or part thereof from these donors may have suffered at least some degree of anoxia, liver from cadaveric donors can be used for obtaining liver-derived cells in cell culture conditions, for instance within about 1 hour, 3 hours, 6 hours, 12 hours, 24 hours or more after the donor's circulation ceased.

"Metabolic defects" in the context of adult liver with metabolic defects, relates to livers from donors suffering of "metabolic liver diseases", preferably noncirrhotic inherited metabolic liver diseases. These livers are anatomically and functionally normal except for some enzyme defects. Cirrhosis is a late-stage scarring of the liver that can result from various chronic liver diseases, but in non-cirrhotic inherited metabolic liver diseases, cirrhosis is not a prominent feature. Instead, these conditions primarily involve disturbances in specific metabolic pathways within the liver. The group of metabolic liver diseases is generally known from literature and by the person skilled in the art, and said person can easily assess whether a specific disease or condition can be considered a metabolic liver disease. Non-limiting examples of literature discussing such diseases are Hansen et al., 2008; and Peter T. Clayton, 2002. Said "livers with metabolic defects" are thus preferably noncirrhotic livers, that are healthy aside from having said metabolic defect(s), i.e., said defect(s) in one or more enzymes. Said livers preferably show at most some mild fibrosis, i.e scarring. The term "fibrosis" as used herein refers to the formation of excess fibrous connective tissue in an organ or tissue in a reparative or reactive process. The term "liver fibrosis" refers to the accumulation of interstitial or "scar" extracellular matrix after either acute or chronic liver injury. Cirrhosis, the end-stage of progressive fibrosis, is characterized by septum formation and rings of scar that surround nodules of hepatocytes. Typically, fibrosis requires years or decades to become clinically apparent, but notable exceptions in which cirrhosis develops over months may include pediatric liver disease (e.g., biliary atresia), drug-induced liver disease, and viral hepatitis associated with immunosuppression after LT. The livers used in the present invention are thus preferably at most showing some mild fibrosis, without this having resulted in actual cirrhosis.

Said genetic mutations and/or enzymatic deficiencies thus can result in a metabolic liver disease or disorder.

As non-limiting examples, metabolic liver diseases include familial amyloidotic polyneuropathy (FAP), maple syrup urine disease (MSUD), acute intermittent porphyria, hereditary fibrinogen A o-chain amyloidosis, propionic acidemia, hyperhomocysteinemia, methylmalonic acidemia, familial hypercholesterolemia, primary hyperoxaluria, hemophilia A, urea cycle disorders, and Crigler Najjar syndrome.

"Amyloidotic Polyneuropathy (FAP)" is a group of inherited disorders characterized by the abnormal accumulation of amyloid proteins in various tissues, including the liver and peripheral nerves. "Maple Syrup Urine Disease (MSUD)" is a genetic disorder that affects the metabolism of branched-chain amino acids (leucine, isoleucine, and valine). If untreated, it can cause severe neurological and liver complications. "Acute Intermittent Porphyria (AIP)" is a type of hepatic porphyria, where metabolic defects in the liver lead to the accumulation of certain porphyrin precursors. "Hereditary Fibrinogen A o-Chain Amyloidosis" is a type of systemic amyloidosis caused by mutations in the fibrinogen A o-chain gene. Amyloid deposits can accumulate in various organs, including the liver. "Propionic Acidemia" is an inborn error of metabolism in which the body cannot properly process certain amino acids and odd-chain fatty acids. It can lead to metabolic acidosis and liver dysfunction. "Hyperhomocysteinemia" is a condition characterized by elevated levels of homocysteine in the blood, which can be caused by genetic or nutritional factors. Severe cases can lead to liver problems. "Methylmalonic Acidemia" is another inborn error of metabolism that affects the breakdown of certain amino acids and fatty acids, leading to the accumulation of toxic metabolites and potential liver involvement. "Familial Hypercholesterolemia" is a genetic disorder that leads to high levels of cholesterol in the blood, and if left untreated, it can lead to fatty deposits in the liver and other complications. "Primary Hyperoxaluria" is a group of rare genetic disorders that cause the overproduction of oxalate, leading to the formation of kidney stones and, in some cases, liver problems. "Hemophilia A" is primarily a bleeding disorder caused by a deficiency of clotting factor VIII. As the liver is the main site of factor VIII synthesis, liver disease can have important implications for the management of hemophilia A. "Urea cycle disorders" are inborn errors of metabolism resulting from defects in one of the enzymes or transporter molecules

involved in the hepatic removal of ammonia from the bloodstream. Removal of ammonia from the bloodstream normally occurs via its conversion to urea, which is then excreted by the kidneys. "Crigler Najjar syndrome" is a rare autosomal recessive inherited disorder characterized by the absence or decreased activity of UDP-glucuronosyltransferase, an enzyme required for glucuronidation of unconjugated bilirubin in the liver. It is one of the major causes of congenital non-hemolytic jaundice.

In one embodiment, said adult livers with metabolic defects or part thereof are adult livers of part thereof displaying impaired metabolic functions linked to genetic mutations and/or enzymatic deficiencies resulting in a metabolic liver diseases or disorder, preferably chosen from the group of familial amyloidotic polyneuropathy (FAP), maple syrup urine disease (MSUD), acute intermittent porphyria, hereditary fibrinogen A o-chain amyloidosis, propionic acidemia, hyperhomocysteinemia, methylmalonic acidemia, familial hypercholesterolemia, primary hyperoxaluria, hemophilia A, urea cycle disorders, and Crigler Najjar syndrome.

"Isolated adult livers with metabolic defects or a part thereof" refers to one or more isolated adult livers with one or more metabolic defects or a part thereof, in particular one or more isolated adult livers with one metabolic defect or a part thereof, more particularly one isolated adult liver with one metabolic defect or a part thereof.

In an embodiment, the method comprises a step of screening said donor for the presence of metabolic liver diseases or disorders.

In another or further embodiment, the method comprises a step of screening said adult liver or part thereof for the presence of metabolic defects.

In a preferred embodiment, the liver with metabolic defects or a part thereof shows an unaltered structure compared to such liver or part thereof without said metabolic defects. This includes but is not limited to livers without clear signs of cirrhosis. Optionally, some mild fibrosis can be allowed.

In an embodiment, the population of cells obtained by the method comprises at least 60%, or between 60% and 99%, or between 70% and 90% of isolated liver-derived cells.

In an embodiment, said liver-derived cells are chosen from liver progenitor cells such as adult liver progenitor cells, hepatocyte progenitors, cholangiocyte progenitors, stellate cell progenitors, liver endothelial cell progenitors, and/or Kupffer cell progenitors.

"Liver Progenitor Cells" are precursor cells in the liver that can give rise to hepatocytes and other liver cell types. They have regenerative potential. "Hepatocytes" are cells of the main parenchymal tissue of the liver, making up 80% of the liver's mass. "Cholangiocytes" are cells that line the bile ducts in the liver. They play a role in bile production and transport. "Liver stellate cells" or "hepatic stellate cells" are the main producers and maintainers of the basal extracellular matrix and serve as the primary storage pools of vitamin in the body. They are also involved in liver fibrosis and tissue repair. Liver "endothelial cells" form the blood vessels within the liver. They are crucial for maintaining blood flow and can be relevant in vascular and liver tissue engineering applications. "Kupffer Cells" are specialized macrophages located in the liver. They play a role in immune responses in the liver and can be relevant in immunotherapy approaches.

The nature of said cells can be confirmed by proteomic techniques as is known by a person skilled in the art, such as by comparing the marker profile of said cells to a known marker profile of any of said adult liver progenitor cells, hepatocyte progenitors, cholangiocyte progenitors, stellate cell progenitors, liver endothelial cell progenitors, and/or Kupffer cell progenitors.

Each cell type, including progenitors of liver cells, hepatocytes, cholangiocytes, stellate cells, liver endothelial cells, and Kupffer cells, has a unique protein marker profile. These markers can include specific proteins that are expressed at varying levels in different cell types. By using techniques like mass spectrometry and Western blotting, researchers can analyze the protein composition of a cell and compare it to known marker profiles to confirm the cell's identity. Further, mass spectrometry is a powerful technique that can be used to identify and quantify proteins in a sample. By analyzing the mass-to-charge ratios of peptides generated from protein digestion, researchers can determine the presence and abundance of specific proteins associated with particular cell types. This method can help confirm the identity of the cells. Also, immunohistochemistry (IHC) can be used. It involves using specific antibodies that bind to particular proteins. By staining cell samples with antibodies against known markers for each cell type, researchers can visually confirm the presence of these markers in the cells of interest. This technique is often used for tissue sections and can provide spatial information about protein expression. Moreover, RNA analysis can be used. While proteomic techniques directly assess protein expression, gene expression analysis through techniques like RNA sequencing (RNA-seq) can also be used to confirm the identity of cells. Different cell types have distinct gene expression profiles, and comparing the RNA expression patterns of cells to known profiles can help identify them. In addition, flow cytometry, a method that can be used to analyze the protein expression in the individual cells of a suspension, can help distinguish and quantify different cell populations based on their protein markers.

Alternatives to proteomic techniques for confirming the identity of cells may include: Morphological Examination: Visual inspection of cell morphology under a microscope can provide clues about cell type. Different cell types often have distinct shapes and structures. Functional Assays specific to certain cell types, for example, hepatocytes are known for their ability to metabolize drugs, so drug metabolism assays can confirm their identity. Genetic Analysis: in addition to RNA analysis, genetic markers specific to certain cell types can be investigated using techniques such as polymerase chain reaction (PCR) or in situ hybridization.

In a preferred embodiment, said liver-derived cells are adult liver progenitor cells.

In an embodiment, said liver-derived cells are derived from isolated hepatic parenchymal and non-parenchymal fractions of said isolated adult livers with metabolic defects.

The "hepatic parenchymal fraction" refers to the portion of an isolated adult liver primarily composed of hepatocytes, which are the main functional cells responsible for metabolic processes in the liver such as detoxification, metabolism of nutrients, and synthesis of proteins. This parenchymal fraction includes the tissue rich in hepatocytes and potentially their progenitors.

The "non-parenchymal fraction" of an isolated adult liver includes all the liver tissue components other than hepatocytes. This fraction encompasses a diverse range of cell types and structures found in the liver, such as liver endothelial cells, Kupffer cells, stellate cells, cholangiocytes, their progenitors, and various connective tissues. It also includes components like blood vessels, bile ducts, and other immune cells.

The non-parenchymal fraction plays critical roles in supporting liver functions, including immune responses, tissue repair, and structural integrity.

In an embodiment, the liver-derived cells are adult liver progenitor cells, and the method further comprises establishing that said adult liver progenitor cells express at least one mesenchymal marker selected from CD90, CD44, CD73, CD13, CD140b, CD29, vimentin and alpha-smooth muscle actin (ASMA), and optionally expressing at least one hepatic marker and/or exhibiting at least one liver-specific activity.

In an embodiment, the adult liver progenitor cells are measured positive for at least one hepatic marker selected from HNF-3B, HNF-4, CYP1A2, CYP2C9, CYP2E1 and CYP3A4.

In an embodiment, the adult liver progenitor cells exhibit at least one liver-specific activity selected from urea secretion, bilirubin conjugation, alpha-1-antitrypsin secretion, and CYP3A4 activity.

In another preferred embodiment, the adult liver progenitor cells are characterized in that they express, or are positive for one or more of CD90, CD73, vimentin and ASMA, preferably for each of CD90, CD73, vimentin and ASMA.

In yet another embodiment, the adult liver progenitor cells are positive for CD90, CD73, vimentin and ASMA, and that exhibit in average less than about 2.5% clonal aberrations per metaphase and/or less than about 15% non-clonal aberrations per metaphase.

In an embodiment, the adult liver progenitor cells secrete hepatocyte growth factor (HGF), and/or prostaglandin E2 (PGE2).

In an embodiment, the liver-derived cells are adult liver progenitor cells, more particularly said cells are human adult liver-derived progenitor cells (HALPC) having an elongated shape and mesenchymal morphology.

In one embodiment, the liver-derived cells are for allogeneic use.

In an embodiment, the method comprises:
a) disassociating adult liver with metabolic defects or a part thereof to form a population of primary liver cells having said metabolic defects;
b) generating a preparation of the population of primary liver cells of step a) thereby obtaining a suspension of primary liver cells;
c) culturing and expanding said suspension of primary liver cells of step b); and
d) harvesting said cultured and expanded cell population of step c).

Concerning step a) of the method, the dissociation step involves obtaining adult liver with metabolic defects or a part thereof that contains, together with fully differentiated hepatocytes, an amount of primary cells that can be used for producing the various isolated liver-derived cells of invention. The liver primary cells are preferentially isolated from human liver tissues which can be obtained from adult liver with said metabolic defects.

The tissues (from surgically resected liver samples or liver biopsies) that are harvested as indicated above may be cooled to about room temperature, or to a temperature lower than room temperature, but usually freezing of the tissue or parts thereof is avoided, esp. where such freezing would result in nucleation or ice crystal growth. For example, the tissue may be kept at any temperature between about 1°C or about 4°C and room temperature, and may advantageously be kept at about 4°C, e.g. on ice. The tissue may be cooled for all or part of the ischemic time, i.e., the time after cessation of circulation in the donor. That is, the tissue can be subjected to warm ischemia, cold ischemia, or a combination of warm and cold ischemia. The harvested tissue may be so kept for, e.g., up to 48 hours before processing, preferably for less than 24 hours, e.g., more preferably for less than 12 hours (e.g., less than 6, 3, or 1 hour). The harvested tissue may advantageously be but need not be kept in, e.g., completely or at least partly submerged in, a suitable medium and/or may be but need not be perfused with the suitable medium, before further processing of the tissue. A skilled person is able to select a suitable medium which can support the survival of the cells of the tissue during the period before processing.

The method of the invention comprises disassociating adult liver tissue as described above to obtain a population of primary cells. The term "disassociating" as used herein generally refers to partly or completely disrupting the cellular organization of a tissue or organ, i.e., partly or completely disrupting the association between cells and cellular components of a tissue or organ, to obtain a suspension of cells (a cell population) from the said tissue or organ. The suspension may comprise solitary or single cells, as well as cells physically attached to form clusters or clumps of two or more cells. Disassociating preferably does not cause or causes as small as possible reduction in cell viability. A suitable method for disassociating liver or part thereof to obtain a population (suspension) of primary cells therefrom may be any method well known in the art, including but not limited to, enzymatic digestion, mechanical separation, filtration, centrifugation and combinations thereof. In particular, the method for disassociating liver or part thereof may comprise enzymatic digestion of the liver tissue to release liver cells and/or mechanical disruption or separation of the liver tissue to release liver cells. Small, thin fragments of liver tissues that are obtained by a liver biopsy may be used directly for pursuing cell culture according to the following step c) without enzymatic or mechanical disruption.

Methods for disassociating liver or part thereof as above are documented in the literature as the widely used collagenase perfusion technique in two or more steps, which has been variously adapted and modified for performing it with whole livers or segments of liver. The liver tissue is perfused with a divalent cation-free buffer solution, preheated at 37°C, containing a cation-chelating agent (e.g., EDTA or EGTA). Buffer solutions can comprise salt solutions (e.g., HEPES, Williams E medium) or any other balanced salt solution that can also include salts such as NaCl and KCl, among others. This leads to disruption of the desmosomal structures that hold cells together. The tissue is then perfused with the buffer solution containing divalent cation(s), such as Ca²⁺ and Mg²⁺, and matrix-degrading enzymes that act to digest the tissue.

The primary liver cells are usually released by gentle mechanical disruption and/or passing through filters, to mechanically complete the cell dissociation process. Such filters may have sieve sizes that allow passage of cells through about 0.1mm, 0.25mm, 0.50mm, 1mm or more. A succession of filters with progressively smaller sieve sizes may be used to gradually disassociate the tissue and release cells. The dissociated cells are rinsed with a buffer containing protease inhibitor, serum and/or plasma to inactivate collagenase and other enzymes used in the perfusion process, and then separated from the mixture by pelleting them with low-speed centrifugation (e.g, at between 10 x g and 500 x g). Most of, if not all, viable cells can be pelleted, while dead cells and cell debris are substantially eliminated and subsequently are washed with ice-cold buffer solution to purify the cell suspension. The number and quality of the primary liver cells can vary depending on the quality of the tissue, the compositions of different solutions that are used, and the type and concentration of enzyme. The enzyme is frequently collagenase but also pronase, trypsin, hyaluronidase, thermolysin, and combinations thereof can be used. Collagenase may consist of a poorly purified blend of enzymes and/or exhibit protease activity, which may cause unwanted reactions affecting the quality and quantity of viable cells that can in turn be avoided by selecting enzyme preparations of sufficient purity and quality. Other methods of harvesting primary liver cells may exclude enzymatic digestion techniques and may involve perfusing liver with solutions containing sucrose followed by mechanical disruption.

An example protocol for the isolation of primary human liver cells from normal and nonalcoholic steatohepatitis livers is provided in Xiao Liu et al., (2023) and Faccioli et al., (2021).

Concerning step b) of the method, the preparation of liver primary cells that is obtained following the disassociation of liver tissue may typically be a heterogeneous population of primary liver cells, comprising cells belonging to any liver-constituting cell types, including progenitor or stem cells, that may have been present in liver parenchyma and or in non-parenchyma thereof. Exemplary liver-constituting cell types include hepatocytes, cholangiocytes, Kupffer cells, hepatic stellate cells, and liver endothelial cells, in addition to stem or progenitor cells that may be present or originated in liver tissue sample.

The term "hepatocyte" encompasses epithelial, parenchymal liver cells, including but not limited to hepatocytes of different sizes, functionality or ploidy (e.g., diploid, tetraploid, octaploid).

The term "primary cell" includes cells present in a suspension of cells obtained from a tissue or organ of a subject, e.g., liver, by disassociating cells present in such explanted tissue or organ with appropriate techniques.

The methods of the invention may preferably start from a cell population representative of most, if not all, liver cell types at the scope of obtaining the desired liver-derived cells in cell culture conditions. A suitable starting cell population for obtaining the liver-derived cells of the method may comprise hepatocytes in different proportions (0.1%, 1%, 10%, or more of total cells), according to the method of disassociating liver and/or any methods for fractioning or enriching the initial preparation for hepatocytes and/or other cell types on the basis of physical properties (dimension, morphology), viability, cell culture conditions, or cell surface marker expression by applying any suitable techniques.

The population of primary cells as defined and obtained herein by disassociating liver (or part of it) can be used immediately for establishing cell cultures as fresh primary liver cells or, preferably, stored as cryopreserved preparations of primary liver cells using common technologies for their long-term preservation. Indeed, the use of cryopreserved cell preparations appears having a positive effect on the efficiency with which the liver-derived cells are later produced in cell culture. Cells in these samples may be frozen in a cell culture medium or a solution for preserving cells or organs (e. g. Viaspan, Cryostor, Celsior) that is supplemented or not with other compounds such as growth factors, serum, buffer solutions, Glucose, Albumin, ethylene glycol, sucrose, dextrose, DMSO or any other cryoprotectant. Each cryopreserved preparation may contain at least 10³, 10⁴, 10⁵, 10⁶, 10⁷, 10⁸ cells or more per cryovial or bag, at scope of producing and isolating higher amount of liver-derived cells in cell culture conditions after appropriately thawing the sample and, if needed, washing the cells with appropriate buffer or cell culture medium for eliminating residual cell culture medium or a solution for preserving cells or organs.

Step b) preferably results in a suspension of primary liver cells.

In an embodiment, the preparations of the primary liver cells of step b) are cryopreserved.

In an aspect, the present invention also relates to a suspension of primary liver cells obtained from isolated adult livers with metabolic defects or a part thereof. In a further aspect, the present invention also relates to a suspension of primary liver cells obtained from one isolated adult liver with one metabolic defect or a part thereof.

In an embodiment, said primary liver cells have metabolic defects. This can for instance be assessed by further culturing (part of) the cells, and testing these cells for the presence of said metabolic defects.

Said isolated adult livers or part thereof, and said metabolic defects, are as described above or below in any one of the embodiments.

In an embodiment, said suspension is obtained via steps a) and b) of the method for obtaining a population of isolated liver-derived cells suitable for therapeutic use as described above.

In an embodiment, the suspension of primary liver cells is cryopreserved.

The invention therefore also relates to a use of the suspension of primary liver cells as described above in the preparation of a population of isolated liver-derived cells for therapeutic use.

Coming back to the method as described in more detail above in regard to step a) and b), concerning step c), the suspension of primary liver cells of step b) is cultured and expanded.

Alternatively or further, the method may start from step c) using the suspension of primary liver cells of the invention thereby skipping steps a) and b). In this case, the method for obtaining a population of isolated liver-derived cells suitable for therapeutic use comprises steps c) and d), wherein the suspension of primary liver cells of the invention is directly used as starting material.

In an embodiment, culturing the cells in step c) comprises culturing the cells of the suspension of step b) under conditions that cause or allow the emergence of a cell population, followed by culturing the emerged population under conditions that cause or allow their expansion.

Once the cells are cultured and the population has emerged, one can test the cells for the presence of metabolic defects.

In an embodiment, step c) comprises culturing the cells of step b) under conditions that cause or allow the emergence of a population of cells having an elongated shape and mesenchymal morphology, followed by culturing said resulting cells under conditions that cause their expansion.

"Conditions that cause/allow the emergence of a population of cells having an elongated shape and mesenchymal morphology" are generally known by a skilled person. The skilled person can easily verify whether the cells have obtained the required shape and morphology. In addition, also "conditions that cause its/their expansion" are generally known, and can easily be verified.

Expansion of cells refers to the process of increasing the number of cells in culture. This is typically achieved by providing the stem cells with appropriate conditions and nutrients that support their growth and proliferation. The goal is to obtain a larger population of cells while maintaining their characteristics.

Said culturing of steps c) can be directly onto a fully synthetic support (e.g. plastic or any polymeric substance) or a synthetic support pre-coated with feeder cells, protein extracts, or any other material of biological origin that preferably allow the adherence and the proliferation of similar primary cells and the emergence of a population of liver derived cells, such as of adult liver progenitor cells having an elongated shape and mesenchymal morphology.

Preferably cells from the primary cell population that have adhered to the said substrate, are cultured for at least 7 days, preferably at least 10, or at least 12 days. More preferably, the cells from the primary cell population are cultured within 7 and 12 days, to obtain a population of adherent cells that is sufficiently enriched for viable primary cells that can provide the liver derived cells, such as adult liver progenitor cells of the method.

The term "culturing" broadly refers to conditions for the maintenance and/or growth of cells, and in particular of liver-derived cells in culture. Elements such as the support where cells are cultured and allowing cell adhesion (or, when needed, allowing growth of cell clusters in suspension), composition of cell culture medium, density at which the cells are seeded and maintained, the O₂ and CO₂ concentration, may be adapted for culturing liver-derived cells, as detailed below.

The term "liver progenitor cell" refers to an unspecialized and proliferation-competent cell which is produced by culturing cells that are isolated from liver and which or the progeny of which can give rise to at least one relatively more specialized cell type. A liver progenitor cell gives rise to descendants that can differentiate along one or more lineages to produce increasingly more specialized cells (but preferably hepatocytes or hepato-active cells), wherein such descendants may themselves be progenitor cells, or even produce terminally differentiated liver cells (e.g. fully specialized cells, in particular cells presenting morphological and functional features similar to those of primary human hepatocytes).

Given that the liver tissues that are used in the methods of the invention come from adult liver, the cells produced by the present method can be defined as adult liver progenitor cells. The cells may also be hepatocyte progenitors, cholangiocyte progenitors, stellate cell progenitors, liver endothelial cell progenitors, and/or Kupffer cell progenitors as discussed above.

Preferably, the cells are adult liver progenitor cells having an elongated shape and mesenchymal morphology.

Following emergence and proliferation, the cells can be further characterized by technologies that allow detecting relevant markers already at this stage (that is, before or after expanding the cells) as being at least one mesenchymal marker selected from CD90, CD44, CD73, CD13, CD140b, CD29, vimentin and alpha-smooth muscle actin (ASMA), and optionally expressing at least one hepatic marker and/or exhibiting at least one liver-specific activity.

In an embodiment, the method further comprises establishing that said cells of the expanded cell population are human adult liver-derived progenitor cells (HALPC) expressing at least one mesenchymal marker selected from CD90, CD44, CD73, CD13, CD140b, CD29, vimentin and alpha-smooth muscle actin (ASMA), and optionally expressing at least one hepatic marker and/or exhibiting at least one liver-specific activity.

Among the technologies for identifying such markers and measuring them as being positive or negative, immunocytochemistry or analysis of cell culture media are preferred since allowing marker detection even with the low amount of cells that are available at this step, without destroying them (as it would be in the case of Western Blot or Flow Cytometry).

The liver-derived cells emerge from a primary population of liver cells that is plated onto a substrate preferably allowing adherence of cells within an in vitro environment capable of promoting survival and/or growth of such cells. This environment may prevent an undesired exchange of matter between the said environment (i.e. the cell culture container) and the surroundings (e.g. by avoiding contamination of the laboratory environment), while it can allow continuous or intermittent exchange of other, useful, components between culture vessels (e.g. by an occasional exchange of a part or all of the culture medium, the continuous exchange of gases).

The culture vessels can be cell culture flasks, bottles, well plates, multi-tray cell stacks, bioreactors and dishes of various formats, preferably displaying one or more substrate surfaces, including microcarriers, compatible with cell adhesion, such that the plated cells can contact this substrate to be maintained adherent cell cultures. In general, a substrate which allows adherence of cells thereto may be any substantially hydrophilic substrate, being glass or a synthetic polymeric material (such as polycarbonates, polystyrenes, polyorthoesters, polyphosphazenes, polyphosphates, polyesters, nylons or mixtures thereof) that are generally shaped and treated in order to provide hydrophilic substrate surfaces and thereby enhance the likelihood of effective cell attachment. Surface treatment may take the form of a surface coating, or may involve generating chemical groups on the polymer surface that have a general affinity for water or otherwise exhibit sufficient polarity to permit stable adsorption to another polar group. These functional groups lead to hydrophilicity and/or an increase in surface oxygen and are properties recognized to enhance cell growth on so modified substrate surfaces. Such chemical groups may include groups such as amines, amides, carbonyls, carboxylates, esters, hydroxyls, or sulfhydryls that can be also introduced by treating them with specific wave frequency-based technologies. Cell adhesion can be facilitated by coating the treated plastic surfaces with a layer of a suitable matrix. The coating may involve suitable polycations (e.g., polyomithine or polylysine) or, preferably, one or more components of extracellular matrix: fibrin, laminin, non-/fibrous collagens (preferably collagen type 1), glycosaminoglycans (e.g., heparin or heparan sulphate) or proteins such as fibronectin, gelatine, vitronectin, elastin, tenascin, aggrecan, agrin, bone sialoprotein, cartilage matrix protein, fibrinogen, fibulin, mucins, entactin, osteopontin, plasminogen, restrictin, serglycin, osteonectin, versican, thrombospondin 1, or cell adhesion molecules including cadherins, connexins, selectins, by themselves or in various combinations. Preferred examples may include collagen compositions, comprising or not other extracellular matrix components. Alternatively, synthetic peptides that are fragments or otherwise derived from the proteins listed above, gels, molecular scaffolds and other three-dimensional structures that are formed from synthetic and/or biological materials can be used in this scope. The primary cell suspension may be contacted with the adherent surface for a period of time (e.g. at least 2, 4, 6, 12, 24 hours, or more) that is sufficient for allowing the primary liver cell populations to attach to adherent substrate, before removing any non-adherent matter from the culture system (e.g., non-viable or dead cells and cell debris) by discarding medium from the culture system and optionally washing, once or repeatedly, the adherent cells. Then, the culture system is provided with any suitable medium or isotonic buffer (e.g., PBS). Hereby, cells from the primary liver cell population, which have adhered to the surface, are selected for further culturing and may be counted in order to further evaluate the plating density that may be expressed as number of cells plated per cm² of the said surface (e.g. between 10 and 10⁵ cells/cm²).

The suspension of primary liver cells, directly at plating or after washing the cells, is maintained in a liquid medium, which supports their survival and/or growth. The medium may be added to the system before, together with or after the introduction of the cells thereto. The medium may be fresh (i.e., not previously used for culturing of cells) or may comprise at least a fraction which has been conditioned by prior culturing cells of liver origin (or of any other origin) therein. In particular, the medium may be any suitable culture medium for culturing liver progenitor cells as described in the literature and it may be regularly exchanged (e.g., each hour, 3 hours, 12 hours, 24 hours or more) with a fresh medium presenting the same or a different feature (e.g. composition, pH, or oxidative status). The whole volume of the medium may be changed or, alternatively, only part of the medium may be changed, such that a fraction of the medium conditioned by the previous culturing of the cells is retained. Alternatively, the medium is not exchanged until the cells are transferred into another culture vessel, prolonging the culture of the cells in a way that most of the cells not of interest (e.g. hepatocytes and other fully differentiated cells of liver origin) are detached and die, and fresh medium may be simply added regularly.

As understood herein, the cell culture medium is a liquid medium which supports the survival and/or growth of the cells. The liquid culture medium may be added to the culturing environment or system before, together with or after the introduction of the cells thereto. The term "cell medium" or "cell culture medium" or "medium" may generally refer to an aqueous liquid or gelatinous substance comprising nutrients which can be used for maintenance or growth of cells. In the context of the present invention, a liquid medium is used.

The cell culture medium as used in step b) and/or subsequent steps such as step c) may in a specific embodiment be serum-free. As per its conventional definition, "serum" is obtained from a sample of whole blood by first allowing clotting to take place in the sample and subsequently separating the so formed clot and cellular components of the blood sample from the liquid component (serum) by an appropriate technique, typically by centrifugation. The medium used for culturing step b), and also in any one or all of the subsequent steps of the process such as step c) may be free of serum that is derived from any source, whether animal, and/or human. In other words, serum as such is absent. However, this definition does not exclude the presence of an individual component that has been isolated from serum, such as highly purified serum albumin.

The cell culture medium which may be used in step b), and/or subsequent steps of the process such as step c) may be in a particular embodiment, also xeno-free. The term 'xeno-free', as used herein, may be understood as 'animal-component free' (ACF), and refers to a medium (or substrate) which contains no non-human animal-derived or sourced components. In other words, the cell culture medium does not contain any ingredients of animal origin, or ingredients which may have been produced using animal host cells or animal expression cultures. The term 'animal' as used herein is understood to be any non-human animal, e.g. non-human mammals, such as non-human primates, fetal, calf or adult bovine, horse, porcine, lamb, goat, dog, rabbit, mouse or rat.

In one specific embodiment, the culture medium is a chemically-defined medium, meaning that the medium comprises no human purified components derived from human serum, but only recombinant components, which are also preferably manufactured from non-animal expression cultures.

As said, although free of serum as such, the cell culture medium used in step b) of the process, and/or subsequent steps of the process such as step c) may comprise, as an individual component, purified native or recombinant human serum albumin. In one particular embodiment, the cell culture medium as used in step b) of the process according to the invention, and/or subsequent steps of the process such as step c) comprises a recombinant human serum albumin.

The primary cells are preferably cultured in the presence of a liquid culture medium, i.e. the primary liver cell suspension, for growing adherent cells that is based on defined chemical media with addition of bovine, human or other animal serum that, besides providing nutrients and/or growth promoters, may also promote the growth/adherence or the elimination/detachment of specific cell types. Basal media formulations (available, e.g., from the American Type Culture Collection, ATCC; or from Invitrogen, Carlsbad, California) can be used to culture the primary cells herein, including but not limited to Eagle's Minimum Essential Medium (MEM), Dulbecco's Modified Eagle's Medium (DMEM), alpha modified Minimum Essential Medium (alpha-MEM), Basal Medium Essential (BME), Iscove's Modified Dulbecco's Medium (IMDM), BGJb medium, F-12 Nutrient Mixture (Ham), Liebovitz L-15, DMEM/F-12, Essential Modified Eagle's Medium (EMEM), RPMI-1640, Medium 199, Waymouth's MB 752/1 or Williams Medium E, and modifications and/or combinations thereof. Compositions of these basal media and criteria to adapt concentrations of media and/or media supplements as necessary for the cells cultured are generally known. A preferred basal medium formulation may be one of those available commercially such as Williams Medium E, IMDM or DMEM, which are reported to sustain in vitro culture of adult liver cells, and including a mixture of growth factors for their appropriate growth, proliferation, maintenance of the desired markers and/or biological activity, or long-term storage.

Such basal media formulations contain ingredients necessary for mammal cell development, which are known per se such as inorganic salts (in particular salts containing Na, K, Mg, Ca, Cl, P and possibly Cu, Fe, Se and Zn), physiological buffers (e.g., HEPES, bicarbonate), nucleotides, nucleosides and/or nucleic acid bases, ribose, deoxyribose, amino acids, vitamins, anti-oxidants (e.g., glutathione) and sources of carbon (e.g. glucose, pyruvate). Additional supplements can be used to supply the cells with the necessary trace elements and substances for optimal growth and expansion. Such supplements include insulin, transferrin, selenium salts, and combinations thereof. These components can be included in a salt solution such as Hanks' Balanced Salt Solution (HBSS), Earle's Salt Solution. Further anti-oxidant supplements may be added, e.g. β-mercaptoethanol. While many basal media already contain amino acids, some amino acids may be supplemented later, e.g., L-glutamine, which is known to be less stable when in solution. A medium may be further supplied with antibiotic and/or antimycotic compounds, such as, typically, mixtures of penicillin and streptomycin, and/or other compounds. Most importantly, cell culture media can be complemented with mammalian plasma or sera that contain cellular factors and components that are necessary for cell viability and expansion and that, under certain condition, may be replaced with synthetic components.

The term "serum", as conventionally defined, is obtained from a sample of whole blood by first allowing clotting to take place in the sample and subsequently separating the so formed clot and cellular components of the blood sample from the liquid component (serum) by an appropriate technique, typically by centrifugation. An inert catalyst, e.g., glass beads or powder, can facilitate clotting. Advantageously, serum can be prepared using serum-separating vessels (SST), which contain the inert catalyst to mammals.

The serum or plasma may be obtained commercially and from an organism of the same species as is the species from which the primary liver cells are obtained. Human serum or plasma may be used for culturing primary human liver cells. Alternatively, the medium comprises bovine serum or plasma, preferably foetal bovine (calf) serum or plasma, more preferably foetal bovine (calf) serum (FCS or FBS). The medium comprises between about 0.5% and about 40% (v/v) of serum or plasma or serum replacement, preferably between about 5% and 20% (v/v), e.g., between about 5% and 15% (v/v), e.g., about 10% (v/v). A medium for culturing human liver cells may comprise a mixture of human plasma or serum, preferably human serum, and bovine plasma or serum, preferably bovine serum. Prior to storage or use, the plasma or serum can be irradiated (e.g., gamma-irradiated) or heat inactivated. Heat inactivation is used in the art mainly to remove the complement. Heat inactivation typically involves incubating the plasma or serum at 56°C for 30 to 60 minutes, e.g., 30 minutes, with steady mixing, after which the plasma or serum is allowed to gradually cool to ambient temperature. Optionally, the plasma or serum may also be sterilized prior to storage or use (e.g., by filtration through one or more filters with pore size smaller than 1µm) or treated in accordance to any applicable regulatory policy for culturing human cells for therapeutic use.

Ordinary components of basal media (before addition of serum or plasma), e.g., in particular, isotonic saline, buffers, inorganic salts, amino acids, carbon sources, vitamins, anti-oxidants, pH indicators and antibiotics, are not considered growth factors or differentiation factors in the art. On the other hand, serum or plasma is a complex composition possibly comprising one or more such growth factors.

The term "growth factor" as used herein refers to a biologically active substance which influences proliferation, growth, differentiation, survival and/or migration of various cell types, and may effect developmental, morphological and functional changes in an organism, either alone or when modulated by other substances. A growth factor may typically act by binding, as a ligand, to a receptor (e.g., surface or intracellular receptor) present in cells. A growth factor herein may be particularly a proteinaceous entity comprising one or more polypeptide chains. The term "growth factor" encompasses the members of the fibroblast growth factor (FGF) family, bone morphogenic protein (BMP) family, platelet derived growth factor (PDGF) family, transforming growth factor beta (TGF-beta) family, nerve growth factor (NGF) family, epidermal growth factor (EGF) family, insulin related growth factor (IGF) family, hepatocyte growth factor (HGF) family, the interleukin-6 (IL-6) family (e.g. oncostatin M), hematopoietic growth factors (HeGFs), platelet-derived endothelial cell growth factor (PD-ECGF), angiopoietin, vascular endothelial growth factor (VEGF) family, or glucocorticoids. Where the method is used for human liver cells, the growth factor used in the present method may be a human or recombinant growth factor. The use of human and recombinant growth factors in the present method is preferred since such growth factors are expected to elicit a desirable effect on cellular function.

The medium may comprise a combination of serum or plasma with one or more exogenously added growth factors as defined above, preferably at concentrations in which particular growth factors can induce an effect on in vitro cultured cells. For example, the medium may comprise EGF and insulin, or EGF and dexamethasone, or insulin and dexamethasone, or each EGF, insulin and dexamethasone. EGF may be typically used at concentrations between about 0.1ng/ml and 1 µg/ml and preferably between 1ng/ml and 100ng/ml, e.g., at about 25ng/ml; insulin can be typically used at concentrations between about 0.1 µg/ml and 1mg/ml and preferably between about 1 µg/ml and 100µg/ml, e.g., at about 10µg/ml; dexamethasone can be typically used at concentrations between about 0.1 nM and 1µM, preferably between about 1nM and 100nM, e.g., at about 10nM.

Hormones can also be used in cell culture, for example D-aldosterone, diethylstilbestrol (DES), dexamethasone, insulin, estradiol, hydrocortisone, prolactin, progesterone, thyrotropin, thyroxine, L-thyronine. Liver cells can also benefit from culturing with triiodothyronine, α-tocopherol acetate, and glucagon. Lipids and lipid carriers can also be used to supplement cell culture media. Such lipids and carriers can include, but are not limited to cyclodextrin, cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others. Albumin can similarly be used in fatty-acid free formulations.

Morphological and phenotypic features of the liver derived cells may allow obtaining such cells not only when cryopreserved suspensions of primary liver cells have low plating efficiency, but also by testing and/or adapting known technologies for preparing adherent cells from heterogeneous preparations of primary cells by selecting and combining different technologies, conditions, and/or materials (e.g. the synthetic polymeric material, the component(s) of extracellular matrix, the cell culture medium, the amount or oxygen and/or CO₂ in the incubator, the washing buffer, etc.). In particular, culturing in hypoxic conditions (as obtained by adding an anti-oxidant compound at millimolar or lower concentrations), together with one or more combinations of these other elements can be applied in order to obtain the liver-derived cells in greater amount and/or more quickly from cell culture.

Further or in an alternative embodiment, said culturing of step c) comprises the use of a bioreactor in combination with a microcarrier. The microcarrier serves as a substrate on which the cells may adhere and proliferate.

Bioreactors are cell culture systems providing a controlled microenvironment and suitable conditions which are adapted for the proliferation and expansion of cells in a reproducible, standardized manner. Compared to static or traditional mono-layer cultures, such as '2D' planar culture systems (for example T-flasks), bioreactors are scalable and can be used for larger scale or even industrial scale manufacture of cells.

Preferably, the bioreactor is used in combination with microcarriers which function as substrates and provide surface area for the cells undergoing culturing in step c). The microcarrier may be selected from any one of the embodiments described herein.

In one embodiment, the bioreactor is a stirred tank bioreactor. A stirred tank bioreactor may comprise an impeller system for homogenous stirring and agitation of the cell suspension during culture and expansion. In preferred embodiments, the bioreactor is a horizontal-stirred tank bioreactor or a vertical stirred tank reactor, respectively. A horizontal stirred tank bioreactor usually has an impeller system configured to rotate about a vertical axis, for example by means of impeller blades mounted on a vertically-oriented axle or shaft, generally moving fluid, i.e. culture medium, in an upwards or downwards direction. An example of a horizontal stirred tank type of bioreactor is a spinner flask or spinner reactor, sometimes simply referred to as "spinner". A vertical stirred tank reactor, on the other hand, is configured with a stirrer or impeller system that may be mounted on a horizontally-oriented axle or shaft such as to rotate along a horizontal axis. In one embodiment, the vertical stirred tank reactor is a vertical wheel reactor, in which the impeller system is in the form of a mixing wheel. An example of a vertical stirred tank reactor are the PBS vertical wheel reactors (PBS Biotech, Inc). Other examples of commercially available bioreactors which may be useful or exemplary in the context of the present invention include PADReactor^{®} or PADReactor^{®} Mini, Allegro STR (Pall Corp.), ambr^{®} 250, UniVessel^{®}, Biostat STR ^{®}, (Sartorius-Stedim) and Mobius^{®} STR (Merck-Millipore).

In an alternative embodiment, the bioreactor is not a stirred tank reactor, but a reactor comprising an alternative agitation means to an impeller, for example, a rocking-motion or rocking bioreactor, which is a bioreactor integrated or adapted with a rocking platform which in motion provides wave motion or turbulence to the cell culture.

The bioreactor used may comprise of at least one container, or vessel adapted for containing or holding a cell culture; a stirring or agitation means; and one or more ports, which may optionally be integrated or part of the container or vessel, and which are adapted for the input and/or output of, for example, introducing or replenishing fluids such as culture media, or gases, or for introducing microcarriers, cells, nutrient components, or adapted for harvest, or washing of cells. Preferably, all apparatus or components of the bioreactor and introduced to the bioreactor are sterile, and handled under aseptic conditions.

In the context of the present invention, the term 'microcarrier' refers to a particulate substrate to which cells may adhere or anchor during culture. Microcarriers may alternatively be referred to as 'microbeads', or 'microspheres'. Microcarriers are generally small, roughly spherical particles or beads, usually with a volume median diameter of between 90 to 400 µm. The expression 'microcarrier', as used herein, may refer to a single bead or to a plurality of beads.

Microcarriers are added to a cell culture medium as a growth surface for adhesion-dependent cells. In particular, microcarriers provide a higher surface to volume ratio compared to monolayer cell cultures and may allow for a better process control. Potentially suitable microcarriers may be based on synthetic polymers, such as polystyrene, polyethylene, polyvinyl alcohol (PVA), polylactide, polyglycolide, polycaprolactone, or copolymers of polylactide, polyglycolide, and polycaprolactone, such as poly(lactic-co-glycolic acid) (PLGA).

In one embodiment, microcarriers based on synthetic polymers are used which are substantially non-porous. In another embodiment, the microcarrier is based on a natural polymer, or an optionally modified biopolymer, such as a polymer comprising a polypeptide or polysaccharide. Suitable examples include, without limitation, a microcarrier based on gelatine, alginate, collagen, cross-linked polygalacturonic acid (PGA), crosslinked dextran, glycosaminoglycan, cellulose, or derivatives (such as ethers or esters) of cellulose. Optionally, the microcarrier based on an optionally modified biopolymer is porous. In another embodiment, the microcarrier may be a digestible, or biodegradable microcarrier. In one embodiment, the microcarrier used according to the process of the present invention is a digestible microcarrier, for example a Ca2+ cross-linked polygalacturonic acid (PGA) polymer microcarrier, which may be digested after the harvest stage by treatment with EDTA and pectinase.

In a preferred embodiment, the microcarrier is selected from a polystyrene, gelatin, cross-linked dextran, cross-linked cellulose, polyethylene with silica, polyvinyl alcohol (PVA) and a digestible microcarrier.

The microcarrier, i.e. the surface of the microbeads that the microcarrier consists of, may be coated with a biocompatible material that facilitates the adhesion of the cells. In particular, microcarriers based on synthetic polymers preferably exhibit a coating, but also for certain biopolymer-based microcarriers a coating may be useful to further enhance the properties of the beads in the context of the process of the invention.

In a preferred embodiment, the microcarrier and/or its coating which is utilized in the context of the present invention is xeno-free, i.e. free of any non-human, animal components.

Preferably cells from the primary cell population that have adhered to microcarriers as substrate, are cultured for between 5 and 8 days, preferably 6 to 7 days in a bioreactor, to obtain a population of adherent cells that is sufficiently enriched for viable primary cells that can provide the liver derived cells, such as adult liver progenitor cells of the method.

This step of culturing of the primary liver cells as defined above leads to emergence and proliferation of the liver derived cells such as adult liver progenitor cells, hepatocyte progenitors, cholangiocyte progenitors, stellate cell progenitors, liver endothelial cell progenitors, and/or Kupffer cell progenitors and preferably adult liver progenitor cells in the culture and can be continued until the liver derived cells have proliferated and/or expanded sufficiently.

For example, the said culturing can be continued until the cell population achieved a certain degree of confluence (e.g., at least 50%, 70%, or at least 90% or more confluent). The term "confluence" as used herein refers to a density of cultured cells in which the cells contact one another, covering substantially all of the surfaces available for growth (i.e., fully confluent).

The aim of expanding the cells, i.e. under conditions that causes their expansion, is to progressively enrich for the desired cell population, thus for the adult liver progenitor cells, hepatocyte progenitors, cholangiocyte progenitors, stellate cell progenitors, liver endothelial cell progenitors, and/or Kupffer cell progenitors.

In an embodiment, the cells of the expanded cell population of step c) are expressing at least one mesenchymal marker selected from CD90, CD44, CD73, CD13, CD140b, CD29, vimentin and alpha-smooth muscle actin (ASMA), and optionally expressing at least one hepatic marker and/or exhibiting at least one liver-specific activity.

In an embodiment, the cells of the expanded cell population of step c) are measured positive for at least one hepatic marker selected from HNF-3B, HNF-4, CYP1A2, CYP2C9, CYP2E1 and CYP3A4.

In an embodiment, the cells of the expanded cell population of step c) exhibit at least one liver-specific activity selected from urea secretion, bilirubin conjugation, alpha-1-antitrypsin secretion, and CYP3A4 activity.

In a preferred embodiment, the cells of the population as obtained in step c) or d) of the method according to the invention are characterized in that they express, or are positive for one or more of CD90, CD73, vimentin and ASMA, preferably for each of CD90, CD73, vimentin and ASMA.

In an embodiment, the cells of the population as obtained in step c) or d) of the method according to the invention that are positive for CD90, CD73, vimentin and ASMA, and that exhibit in average less than about 2.5% clonal aberrations per metaphase and/or less than about 15% non-clonal aberrations per metaphase.

In an embodiment, the cells of the population as obtained in step c) or d) of the method according to the invention secrete hepatocyte growth factor (HGF), and/or prostaglandin E2 (PGE2).

In step d), the cell population is harvested or isolated.

Such isolation of population of liver-derived cells applies to cells that have maintained the preferred shape and morphology, i.e. for example elongated shape and mesenchymal morphology, that express the required markers, for example selected from CD90, CD44, CD73, CD13, CD140b, CD29, vimentin and alpha-smooth muscle actin (ASMA), and optionally express at least one hepatic marker and/or exhibit at least one liver-specific activity, further validating the criteria for initially identifying the desire population at step c) above but that can be more easily established given the higher amount of cells that are available after passaging.

Preferably, cells having an elongated shape and mesenchymal morphology, that express markers selected from CD90, CD44, CD73, CD13, CD140b, CD29, vimentin and alpha-smooth muscle actin (ASMA), and optionally express at least one hepatic marker and/or exhibit at least one liver-specific activity, are HALPCs.

In an embodiment, the cells of the harvested cell population are measured positive for at least one hepatic marker selected from HNF-3B, HNF-4, CYP1A2, CYP2C9, CYP2E1 and CYP3A4.

In an embodiment, the cells of the harvested cell population exhibit at least one liver-specific activity selected from urea secretion, bilirubin conjugation, alpha-1-anti-trypsin secretion, and CYP3A4 activity.

The terms "isolating" or "isolation" refers to both the physical identification and the isolation of a cell population from a cell culture or a biological sample that can be performed by applying appropriate cell biology technologies that are either based on the inspection of cell cultures and on the characterization (and physical separation when possible and desired) of cells corresponding to the criteria, or on the automated sorting of cells according to the presence/absence of antigens and/or cell size (such as by FACS). In some embodiments, the terms "isolating" or "isolation" may comprise a further step of physical separation and/or quantification of the cells, especially by carrying out flow cytometry.

The terms "cell population" and "population of cells" refer generally to a group of cells. Unless indicated otherwise, the term refers to a cell group consisting essentially of or comprising cells as defined herein. A cell population may consist essentially of cells having a common phenotype or may comprise at least a fraction of cells having a common phenotype. Cells are said to have a common phenotype when they are substantially similar or identical in one or more demonstrable characteristics, including but not limited to morphological appearance, the level of expression of particular cellular components or products (e.g., RNA or proteins), activity of certain biochemical pathways, proliferation capacity and/or kinetics, differentiation potential and/or response to differentiation signals or behavior during in vitro cultivation (e.g., adherence or monolayer growth). Such demonstrable characteristics may therefore define a cell population or a fraction thereof. A cell population may be "substantially homogeneous" if a substantial majority of cells have a common phenotype. A "substantially homogeneous" cell population may comprise at least 60%, e.g., at least 70%, at least 80%, at least 90%, at least 95%, or even at least 99% of cells having a common phenotype, such as the phenotype specifically referred to the liver-derived cells of the method, or the human adult liver-derived progenitor cells (HALPC). Moreover, a cell population may consist essentially of cells having a common phenotype such as the phenotype of the liver-derived cells if any other cells present in the population do not alter or have a material effect on the overall properties of the cell population and therefore it can be defined as a cell line.

In general, any technology for identifying and characterizing cellular markers for a specific cell type (e.g. mesenchymal, hepatic, hematopoietic, epithelial, endothelial markers) or having a specific localization (e.g. intracellular, cell surface, or secreted) that are published in the literature may be considered appropriate for characterizing the present cells. Such technologies may be grouped in two categories: those that allow maintaining cell integrity during the analysis, and those based on extracts (comprising proteins, nucleic acids, membranes, etc.) that are generated using such cells. When aiming to target markers intracellularly, cells will need to be permeabilized to ensure the accessibility of intracellular components.

At the protein level, technologies such as flow cytometry, FACS, or immunocytochemistry, allow determining the presence/absence of surface or intracellular proteins in the cells by using antibodies or other protein-specific reagents. Flow cytometry is a preferred technology for characterizing cell populations according to the combined presence/absence of surface, or intracellular markers, as determined by single or multiple staining techniques, and/or size and granularity evaluation. Immunocytochemistry also provides relevant information regarding the morphological features that are associated to the combined presence/absence of surface, cytoskeletal, and/or other intracellular markers.

In particular, the presence of at least one mesenchymal marker and/or of at least one hepatic marker should be measured by flow cytometry, immunocytochemistry, or any other technique (generally making use of antibodies, lectins, or other proteins and not requiring the protein or nucleic acid extraction) that allows evaluating the percentage of cells presenting the antigen/protein. The positivity for additional cell surface markers other than those strictly associated to hepatic or mesenchymal features can be similarly measured. Positivity by flow cytometry and immunocytochemistry is preferably defined when at least 60% of cells present the desired marker or receptor. Similarly, the negativity by flow cytometry and immunocytochemistry is preferably defined when less than 20% of cells present the given marker or receptor. In some embodiments, less than 10% of cells present a given negative marker.

In some embodiments, when measuring a given marker, the agent that is used for detection of a marker as defined above or a cell surface protein is immobilized on a solid phase (e.g. a bead, a plate, or a biomaterial), labeled (e.g. fluorescently labeled), and/or recognized by another compound that is labeled (e.g. a secondary antibody). There are numerous methods by which the label can produce a signal detectable by external means, for example, desirably by visual examination or by electromagnetic radiation, heat, and chemical reagents. The label or other signal producing system component can also be bound to a specific binding partner, another molecule or to a support such as beads, using any method known in the art, such as chemically cross-linking or using the biotin- streptavidin system. The label can directly produce a signal, and therefore, additional components are not required to produce a signal. Numerous organic molecules, for example fluorochromes (such as FITC, PE, PC5, PC7, APC, or any other known to be compatible with flow cytometry), absorb ultraviolet and visible light. Other types of label directly produce a signal, such as radioactive isotopes and dyes. Alternatively, the label may need other components to produce a signal, and the signal producing system would then include all the components required to produce a measurable signal, which may include substrates, coenzymes, metal ions, or substances that react with enzymatic products (e.g. chemiluminescent detection of Horseradish Peroxidase).

The liver-specific metabolic activities of the cells comprise biological activities generally associated with liver cells (and to hepatocytes in particular) and that distinguish liver cells from cells present in other tissues, and in particular comprise activities involving binding, activation, and/or degradation of proteins or other substrates as described in the literature and in the Examples. These biological activities are established on the basis of the detection of liver-specific metabolic activities that can be protein/drug binding activities and, more preferably, enzymatic activities on given substrates, or in association to liver-specific molecules that are detected by blotting technologies (Western, or Northern blot), sequencing, isoelectrofocusing, ELISA, or of the internalization of synthetic or natural compounds known to be specifically transported and metabolized within liver cells. Other relevant enzymatic activities other than those strictly associated to hepatic features can be similarly measured and compared with those measured within hepatocytes or other cell types using techniques that are described in the literature.

At the nucleic acid level, whole genome sequencing, PCR, or RT-qPCR can be used to characterize the cells. Hereby, real time PCR can be used to quantify the expression of the gene under investigation, based on the number of cycles and having it normalized against the cycles obtained for 1 or more endogenous controls. In particular, the RT-PCR reaction can be performed using the cells and appropriate primers and buffers but the number of cycles to obtain a signal should not be superior to 25, 30 or 35 cycles.

At the activity level, the presence of a liver-specific metabolic activity can be measured by any appropriate technique that allows evaluating the presence and/or the level of activity of liver-specific enzymes, but preferably should allow quantifying in vitro the actual enzymatic activity, with a given limit of detection of the specific end-product (as it can be easily established with the support of literature and commercially available products) for measuring CYP450 activities, detoxification, glycogen storage, secretion of Alpha-1-Anti-trypsin or albumin, bile production, thrombopoietin production, angiotensinogen production, conversion of ammonia to urea, cholesterol synthesis, glycogenolysis, glycogenesis and lipogenesis. In particular, the positivity for at least a liver-specific metabolic activity is here defined when the activity is measured as being statistically superior to the limit of detection of the end-product (being at least twice, five times, or ten times more than the limit of detection) or approaching the level of activity of primary hepatocytes (superior, identical or 10%; 25%, 50%, 75%, or 90% lower).

The literature provides extensive description of the technologies for evaluating cytochrome P450 activities in human hepatocytes in vitro, in particular regarding the compounds specifically inducing an enzyme activity and the formats that can be used for performing these experiments (Baudoin R et al., 2012; Gerets HH et al., 2012; Gomez-Lechon MJ et al., 2012; Halladay JS et al., 2012; Hoffmann SA et al., 2012; Lubberstedt M et al., 2011; Smith CM et al., 2012). Among the different inducers, drug metabolism in these cells can be assessed using midazolam, ethoxyresorufin, benzoxyresorufin, bupropion, Phenacetin, Diclofenac, tolbutamide, phenobarbital, rifampicin, caffeine, beta-naphthoflavone, omeprazole, dextromethorphan, 3-methylcholanthrene, repaglinide, or other known cyto/hepatotoxic compounds as probes that are listed in the literature (Bale S et al., 2014). Metabolite detection and quantification can be associated to the activity of hepatic enzymes on specific compounds such as CYP1A2 (by detecting paraxanthine or acetaminophen), CYP3A4 (by detecting 1-OH-midazolam or omeprazole sulfone), CYP2C6 (by detecting HO-Bupropion), CYP2C8 (by detecting hydroxyl-repaglinide), CYP2C9 (by detecting 4'HO-Diclofenac), CYP2C19 (by detecting hydroxy-omeprazole or HO-Mephenytoin), CYP2D6 (by detecting dextrorphan), CYP2E1 (by detecting 6-OH-chlorzoxazone), as well as for other major cytochrome P450 activities such as CYP1A2, CYP2A6, CYP1B1, CYP2B6, CYP3A5, CYP3A7, or CYP7A1 (singularly or in appropriate combinations). Other enzymes whose expression or (preferably) activity can be established in cells are UDP-glucuronosyltransferases (such as UGT1A1, UGT2B4, UGT2B7), sulfotransferases (catalyzing the sulfate conjugation of several pharmacologically important endogenous molecules and xenobiotics), tyrosine transferases, tryptophan-2,3-dioxygenase (TDO2 or TDO), indoleamine-2,3-dioxygenases (IDO1 or IDO2), lysyl oxidase (LOX), glutathione S-transferases (e.g. GSTalpha), multidrug resistance proteins (MDR or MRP-1/-2/-3), liver-specific transporters (such as OATP1B1), and other phase I/II/III biotransformation enzymes. Moreover albumin/urea production and secretion, ammonia metabolism, glycogen storage, bile production, thrombopoietin / angiotensinogen production, and galactose/sorbitol elimination rates can be also observed and compared by applying well established protocols.

When a preparation of the present cells is obtained by the methods of the invention, this cell population can be either maintained and/or propagated in conditions that allow growth and doubling without differentiation. Preferably, they are passaged, as non-differentiated adherent cells (or, as indicated above, three-dimensional cell clusters), no more than 2, no more than 3, no more than 4, or o more than 5 times in culture, so that the number of cell doublings can be evaluated for establishing the most appropriate conditions for further in vivo or in vitro use. After one or more passaging in this status, can be induced to differentiate into hepatocyte-like or hepato-active cells.

In an aspect, the present invention relates to a liver cell population obtained from isolated adult livers with metabolic defects or a part thereof, comprising liver-derived cells obtained by a method as described above in any one of the embodiments, or obtained by culturing and expanding the suspension of primary liver cells as described above and subsequently harvesting said expanded cell population as is described in any of the described embodiments.

In an embodiment, the cell population has the characteristics as described above.

In a preferred embodiment of the liver cell population, the liver-derived cells have a metabolic defect.

In an embodiment, the cell population comprises at least 60% or between 60% and 99% or between 70% and 90% of isolated adult liver progenitor cells, hepatocyte progenitors, cholangiocyte progenitors, stellate cell progenitors, liver endothelial cell progenitors, and/or Kupffer cell progenitors.

In an embodiment, said adult liver progenitor cells, hepatocyte progenitors, cholangiocyte progenitors, stellate cell progenitors, liver endothelial cell progenitors, and/or Kupffer cell progenitors of the cell population have a metabolic defect, preferably the same metabolic defect as the adult liver from which they are derived.

In an embodiment, the metabolic defect is linked to one or more genetic mutations and/or enzymatic deficiencies resulting in a metabolic liver disease or disorder, said liver disease or disorder is preferably chosen from the group of familial amyloidotic polyneuropathy (FAP), maple syrup urine disease (MSUD), acute intermittent porphyria, hereditary fibrinogen A o-chain amyloidosis, propionic acidemia, hyperhomocysteinemia, methylmalonic acidemia, familial hypercholesterolemia, primary hyperoxaluria, hemophilia A, urea cycle disorders, and Crigler Najjar syndrome.

In a preferred embodiment of the liver cell population or of the suspension of primary liver cells as described above in any one of the embodiments, where the cells have metabolic defects, said metabolic defects are linked to one or more genetic mutations and/or enzymatic deficiencies resulting in a metabolic liver disease or disorder, said liver disease or disorder is preferably chosen from the group of familial amyloidotic polyneuropathy (FAP), maple syrup urine disease (MSUD), acute intermittent porphyria, hereditary fibrinogen A o-chain amyloidosis, propionic acidemia, hyperhomocysteinemia, methylmalonic acidemia, familial hypercholesterolemia, primary hyperoxaluria, hemophilia A, urea cycle disorders, and Crigler Najjar syndrome.

In another aspect, the invention also relates to a pharmaceutical composition comprising a therapeutically effective amount of the cell population as described above in any one of the embodiments.

The term "therapeutically effective amount" is an amount that is effective to ameliorate a symptom of a disease or condition. A therapeutically effective amount can be a "prophylactically effective amount" as prophylaxis can be considered a therapy.

The term "treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

A "disease" refers to a pathological or abnormal condition in an organism or a part of an organism that impairs its normal physiological functions. Diseases can be caused by various factors, including genetic mutations, infections, environmental factors, or a combination thereof. Diseases often manifest as specific symptoms, signs, or abnormal laboratory findings. They may affect an individual's health, well-being, and quality of life and can range from mild to severe, acute to chronic. Diseases can affect different systems or organs of the body, leading to a wide variety of medical conditions. Examples of diseases include diabetes, cancer, cardiovascular disease, infectious diseases (e.g., influenza, HIV/AIDS), and autoimmune disorders (e.g., rheumatoid arthritis).

A "condition" refers to a state of health or a particular physiological or pathological state that an individual or organism may experience. Conditions can encompass a broad range of health states, including normal and abnormal states. Conditions can include both diseases and non-disease states. Some conditions may not necessarily impair normal physiological functions or may have a transient or mild impact on health. Others may be benign or represent variations of normal physiology.

In another or further aspect, the invention relates to the cell population as described above in any one of the embodiments, or the pharmaceutical composition as described above in any one of the embodiments, for use as a medicament.

In another aspect, the invention relates to the cell population as described above in any one of the embodiments, or the pharmaceutical composition as described above in any one of the embodiments, for use in treating a disease or condition in a subject.

Preferably, the liver cell population as described above in any one of the embodiments is for allogeneic use.

In an embodiment, said cell population or pharmaceutical composition is administered to said subject.

Preferably, said disease or condition is chosen from any one of the diseases and/or conditions as described in any embodiment of any aspects of the present invention.

In an aspect, the invention relates to the cell population as described above in any one of the embodiments, or the pharmaceutical composition as described above in any one of the embodiments, for use in treating a liver disease or condition in a subject.

A first category of liver diseases or conditions is represented by inborn errors of liver metabolism that can be further distinguished into errors of amino acid metabolism (such as Maple Syrup Urine Disease, Phenylketonurias, Tyrosinemia, Propionic Acidemia, Organic Aciduria, and Urea Cycle Disorders including Argininosuccinic Aciduria, Carbamoyl-Phosphate Synthase I Deficiency, Citrullinemia, Hyperargininemia, and Ornithine Carbamoyltransferase Deficiency), of metal metabolism (such as Wilson's Disease or Hemochromatosis), and of carbohydrate metabolism (such as Glycogen Storage Disease type I / II, fructosemia, or Galactosemias), lysosomal disorders (such as Wolman disease, Niemann Pick disease), peroxisomal disorders (such as Refsum Disease), Familial Hypercholesterolemias and other lipid metabolism disorders, mitochondrial diseases (such as Pyruvate Carboxylase Deficiency), and Hyperbilirubinemia (such as Crigler-Najjar Syndrome, Gilbert Syndrome, or Dubin-Johnson syndrome).

A second category is represented by inherited Blood Coagulation Disorders such as Factor V Deficiency, Factor VII Deficiency, Factor VIII Deficiency, Factor IX Deficiency, Factor XI Deficiency, Factor XIII deficiency and other deficiencies due to the insufficient amount of other coagulation-related factors (including other coagulation factors and fibrinogen alpha/beta/gamma chains) or other proteins specifically expressed and secreted by liver into blood stream (such as albumin).

A third category is represented by other liver diseases not directly associated to deficiencies of coagulation or metabolism and includes progressive familial intrahepatic cholestasis type 1/2/3, alpha-1-Antitrypsin Deficiency, Caroli Disease, defects of liver cell transporters, Porphyrias (such as Acute Intermittent Porphyria), fatty liver and other fibrotic liver diseases (NASH/NAFLD), primary biliary cirrhosis, sclerosing cholangitis, liver degenerative diseases, or acute or chronic liver failure (e.g. post-hepatectomy, fulminant, virally induced, acute-on-chronic liver failure (ACLF)).

In an embodiment, said liver disease or condition is a metabolic liver disease or condition, more preferably a noncirrhotic metabolic liver disease or condition. As non-limiting examples, metabolic liver diseases include familial amyloidotic polyneuropathy (FAP), maple syrup urine disease (MSUD), acute intermittent porphyria, hereditary fibrinogen A o-chain amyloidosis, propionic acidemia, hyperhomocysteinemia, methylmalonic acidemia, familial hypercholesterolemia, primary hyperoxaluria, hemophilia A, urea cycle disorders, and Crigler Najjar syndrome.

Said cells can be used for treating liver diseases, in particular those requiring the permanent (or time-limited) re-establishment of liver function in a subject that, according to the literature, requires liver transplantation, hepatocyte transplantation, or liver regeneration given the loss of liver mass and/or function that is observed and that can be grouped in different categories.

In an embodiment, said liver disease or condition is selected from the group of phenylketonuria and other aminoacidopathies, hemophilia and other clotting factor deficiencies, familial hypercholesterolemia and other lipid metabolism disorders, urea cycle disorders, glycogenosis, galactosemia, fructosemia, tyrosinemia, protein and carbohydrate metabolism deficiencies, organic aciduria, mitochondrial diseases, peroxysomal and lysosomal disorders, protein synthesis abnormalities, defects of liver cell transporters, defects of glycosylation, hepatitis, cirrhosis, acute liver failure, acute liver infections, acute chemical toxicity, chronic liver failure, fulminant liver failure, cholangitis, biliary cirrhosis, Alagille syndrome, alpha-1-antitrypsin deficiency, autoimmune hepatitis, biliary atresia, cancer of the liver including hepatocellular carcinoma (HCC), cystic disease of the liver, gallstones, Gilbert's syndrome, hemochromatosis, hepatitis A, hepatitis B, hepatitis C, and other hepatitis viral infections, porphyria, primary sclerosing cholangitis, Reye's syndrome, sarcoidosis, type 1 glycogen storage disease, Wilson's disease, an inherited Blood Coagulation Disorder, progressive familial intrahepatic cholestasis type 1/2/3, defect of liver cell transporters, fatty liver, fibrotic liver disease, Acute-on-chronic liver failure (ACLF) and non-alcoholic fatty liver disease (NAFLD), nonalcoholic steatohepatitis (NASH), or liver degenerative disease.

In an embodiment, said cell population or pharmaceutical composition is administered to said subject, said subject is suffering from or at risk of developing a metabolic liver disease or disorder resulting in a metabolic liver defect, and wherein the metabolic liver defect of said subject is different from the metabolic defect of said administered cells.

In an embodiment, said cell population or pharmaceutical composition is administered to said subject, and wherein the metabolic defect of said administered cells is absent in said subject.

In an embodiment, said cell population or pharmaceutical composition is administered to said subject, said subject is suffering from or at risk of developing a liver disease or disorder, and wherein the liver disease or disorder to be treated of said subject is not caused by or linked to a metabolic defect similar to that present in said administered cells.

In an aspect, the present invention relates to the liver cell population as described above in any one of the embodiments, or the pharmaceutical composition as described above in any one of the embodiments, for use in modulating or influencing impaired vascular permeability in (cells of) a subject.

The term "vascular permeability" as used herein is understood as the capacity of a blood vessel wall to allow for the flow of small molecules (drugs, nutrients, water, ions) or even whole cells in and out of the vessel.

In an aspect, the present invention relates to the liver cell population as described above in any one of the embodiments, or the pharmaceutical composition as described above in any one of the embodiments, for use in the treatment of diseases and/or conditions caused by increased vascular permeability.

The term "increased vascular permeability", as used herein, implies an increased passage of various molecules through the blood vessel wall, including passage of those molecules which may be the cause of acute and chronic inflammation, cancer, and wound healing. Said increased permeability or even hyperpermeability may be mediated by acute or chronic exposure to vascular permeabilizing agents, particularly vascular permeability factor/vascular endothelial growth factor (VPF/VEGF, VEGF-A).

The term "vascular integrity" as used herein, refers to proper functions of various components of the blood vessel wall which maintain vascular homeostasis. One of the early hallmarks of deteriorating vascular integrity is increased permeability which is predominantly controlled by endothelial junction stability. Selective regulation of vascular permeability is achieved by regulation of the size and state of paracellular gaps and control of the transcellular transport.

In embodiments, said diseases and/or conditions caused by increased vascular permeability are chosen from the group of heart, pulmonary and ischemic diseases, diabetes and ocular diseases, cancer (solid tumors), Clarkson's disease and sepsis in a subject.

The term "heart disease" as used herein, refers to any disorder and/or condition that affects the heart. Said heart diseases may include blood vessel diseases, such as coronary artery disease; heart rhythm problems (arrhythmias); and congenital heart defects, among others. The term "heart disease" is often used interchangeably with the term "cardiovascular disease". Cardiovascular disease generally refers to conditions that involve narrowed or blocked blood vessels that can lead to a heart attack, chest pain (angina) or stroke. Other heart conditions, such as those that affect the heart's muscle, valves or rhythm, also are considered forms of heart disease. The term "pulmonary disease" as used herein, refers to any lung disease which is any problem in the lungs that prevents the lungs from working properly. The term "ischemic disease" as used herein refers any disease and/or condition which is connected to impaired tissue/organ or organ system function, due to decrease of a blood flow and sufficient supplementation of said tissue/organ or organ system with oxygen. The types of ischemic disease include, but are not limited to, coronary heart disease, cerebral or brain ischemia, pulmonary ischemia, renal ischemia and the like. The term "diabetes" as used herein, refers to a group of metabolic disorders in which there are high blood sugar levels over a prolonged period. Symptoms of high blood sugar include frequent urination, increased thirst, and increased hunger. Diabetes can cause many complications, especially if left untreated. Acute complications can include diabetic ketoacidosis, hyperosmolar hyperglycemic state, or death. Serious long-term complications include cardiovascular disease, stroke, chronic kidney disease, foot ulcers, and damage to the eyes. The term "ocular disease" as used herein, refers to disorders and conditions affecting an eye. The term "cancer" as used herein, refers to a group of diseases involving abnormal cell growth with the potential to invade or spread to other parts of the body. The term "solid tumor" as used herein, refers to an abnormal mass of tissue that usually does not contain cysts or liquid areas. Solid tumors may be benign (not cancer), or malignant (cancer). Different types of solid tumors are named for the type of cells that form them. Examples of solid tumors are sarcomas, carcinomas, and lymphomas. Leukemias generally do not form solid tumors. The term "Clarkson's disease" as used herein, refers to an idiopathic systemic capillary leak syndrome (SCLS), a rare disorder characterized by transient episodes of hypotensive shock and anasarca thought to arise from reversible microvascular barrier dysfunction. This potentially fatal disorder is characterized by stereotypic 'attacks' of varying intensity of hypovolemic shock and generalized edema in association with hemoconcentration (as detected by an elevated hematocrit concentration) and hypoalbuminemia, typically occurring in the absence of albuminuria. The term "sepsis" as used herein means a life-threatening organ dysfunction caused by a dysregulated host response to infection. Sepsis is characterized by disseminated inflammatory response elicited by microbial infections. Sepsis commonly leads to a state of immunosuppression characterized by lymphocyte apoptosis and susceptibility to nosocomial infections.

In an embodiment, said preferably diseases and/or conditions are triggered by an infection and/or is sepsis or a sepsis-induced disease or condition, such as sepsis-induced myocardial edema, sepsis-induced acute kidney injury, lung sepsis, or Clarkson's disease.

The term "sepsis-induced" as used herein, refers to all diseases and conditions that occur in a tissue, organ and/or organ system, which are the consequence of sepsis and related events. Such conditions may include, but are not limited to sepsis-induced cardiomyopathy, sepsis-induced coagulopathy, poor organ function, acute kidney injury, lung sepsis, poor organ function and/or insufficient blood flow, and the like. The term "septic cardiomyopathy" or "sepsis-induced myocardial edema" as used herein, means a cardiovascular complication in patients with severe sepsis, characterized by a reversible decrease in systolic and/or diastolic left ventricular (LV) function, associated with left ventricular wall edema during sepsis. The term "cecal ligation and puncture" refers to a disorder which consists on the perforation of the cecum allowing the release of fecal material into the peritoneal cavity to generate an exacerbated immune response induced by polymicrobial infection. The polymicrobial sepsis is generated from fecal spillage after needle puncture. Cecal ligation and puncture mode, represents a gold standard and the most frequently used model to study sepsis, because it closely resembles the progression and characteristics of human sepsis. The term "sepsis induced lung injury" as used herein, means acute lung injury or disorder, which occurs secondary to sepsis. The lung is the organ most often affected by the sepsis related systemic inflammatory response, resulting in acute lung injury or the more severe acute respiratory distress syndrome.

In an aspect, the present invention relates to the liver cell population as described above in any one of the embodiments, or the pharmaceutical composition as described above in any one of the embodiments, for use in the prevention and/or treatment of one of more diseases related to cellular senescence.

As used herein, the term "cellular senescence" can be used interchangeably with the terms "cell senescence", "induced cellular senescence", "induced cell senescence", and "cell aging". Cellular senescence refers to an irreversible cell cycle arrest. Mechanisms of senescence and characteristics of senescent cells are well known in the art (see e.g., Kumari et al., Front Cell Dev Biol, 2021).

In some embodiments, the cellular senescence is induced. In some embodiments, the cellular senescence is induced by a stress signal such as telomeric shortening, DNA damage, oxidative stress, oncogenic activation or metabolic dysfunction, disease or damage, oncogene, therapy, diet, or combinations thereof.

As used herein, the term "metabolic dysfunction" refers to an imbalance in at least one pathway, cycle or process of the metabolism of a cell, tissue, organ or organism. Non limitative examples of metabolic dysfunctions include diabetes, hypercholesterolemia or Gaucher disease.

In embodiments, treatment and/or prevention of cellular senescence has a positive effect on the progression or development of at least one disease, wherein said disease is i) selected from the group comprising or consisting of metabolic, genetic, infectious, toxic and autoimmune liver diseases, chronic biliary diseases, cholestatic diseases, age-related diseases, bone and cartilage disorders, pancreatic diseases, kidney diseases, pulmonary diseases, cardiovascular diseases, metabolic diseases, eye diseases, neurodegenerative diseases, skin diseases, inflammatory diseases and cancer, and/or ii) said one of more diseases related to cellular senescence are selected from the group comprising or consisting of hepatic fibrosis, pre-cirrhotic conditions, cirrhosis including liver and biliary cirrhosis, biliary atresia, Alagille syndrome, progressive familial intrahepatic cholestasis, primary biliary cholangitis, primary sclerosing cholangitis, chronic hepatitis, chronic hepatitis B virus (HBV) infection, chronic hepatitis C virus (HCV) infection, cholestasis, osteoporosis, osteoarthritis, atherosclerosis, cardiac hypertrophy, cardiac fibrosis, cardiomyopathy, thrombosis, cataracts, glaucoma, macular degeneration, chronic obstructive pulmonary disease (COPD), pulmonary fibrosis, renal dysfunction, pancreatic fibrosis, type 2 diabetes, Alzheimer disease, Huntington's disease, Parkinson's disease, dementia, lipodystrophy, sarcopenia, age-related cachexia, skin aging, hepatocellular carcinoma, liver cancer, lobular carcinoma, bile duct cancer, melanoma, lung cancer, and islet cell tumor.

In an aspect, the present invention relates to the liver cell population as described above in any one of the embodiments, or the pharmaceutical composition as described above in any one of the embodiments, for use in the prevention and/or treatment of a patient at risk of or suffering from hepatocellular carcinoma (HCC).

As used herein, the term "hepatocellular carcinoma" or "HCC" refers to the most common type of primary liver cancer in adults, and is the most common cause of death in people with cirrhosis. Hepatocellular carcinoma occurs most often in people with chronic liver diseases, liver injury and inflammation, such as cirrhosis caused by hepatitis B or hepatitis C infection, or linked to exposure to toxins such as alcohol, aflatoxin, or pyrrolizidine alkaloids. Certain diseases, such as hemochromatosis and alpha-1 antitrypsin deficiency, markedly increase the risk of developing HCC. Metabolic syndrome and non-alcoholic steatohepatitis (NASH) are also increasingly recognized as risk factors for HCC.

In an embodiment, said population of cells reduce the growth rate of a hepatocellular carcinoma. Preferably said reduction must be seen as compared to said HCCs not treated with said population of cells.

In some embodiment, multiple administrations of the population of cells are performed on the same patient.

In some embodiments, the population of liver derived cells is administered at a dose of 0.25 to 20 million of cells per kg of body weight. Within the scope of this invention, 0.25 to 20 million of cells per kg of body weight include from 0.25 million, 0.30 million, 0.35 million, 0.40 million, 0.45 million, 0.50 million, 0.55 million, 0.60 million, 0.65 million, 0.70 million, 0.75 million, 0.80 million, 0.90 million, 0.95 million, 1.00 million, 1.10 million, 1.20 million, 1.30 million, 1.40 million, 1.50 million, 1.60 million, 1.70 million, 1.80 million, 1.90 million, 2.00 million, 2.10 million, 2.20 million, 2.30 million, 2.40 million, 2.50 million, 2.60 million, 2.70 million, 2.80 million, 2.90 million, 3.00 million, 3.10 million, 3.20 million, 3.30 million, 3.40 million, 3.50 million, 3.60 million, 3.70 million, 3.80 million, 3.90 million, 4.00 million, 4.10 million, 4.20 million, 4.30 million, 4.40 million, 4.50 million, 4.60 million, 4.70 million, 4.80 million, 4.90 million, 5.00 million, 5.10 million, 5.20 million, 5.30 million, 5.40 million, 5.50 million, 5.60 million, 5.70 million, 5.80 million, 5.90 million, 6.00 million, 6.10 million, 6.20 million, 6.30 million, 6.40 million, 6.50 million, 6.60 million, 6.70 million, 6.80 million, 6.90 million, 7.00 million, 7.10 million, 7.20 million, 7.30 million, 7.40 million, 7.50 million, 7.60 million, 7.70 million, 7.80 million, 7.90 million, 8.00 million, 8.10 million, 8.20 million, 8.30 million, 8.40 million, 8.50 million, 8.60 million, 8.70 million, 8.80 million, 8.90 million, 9.00 million, 9.10 million, 9.20 million, 9.30 million, 9.40 million, 9.50 million, 9.60 million, 9.70 million, 9.80 million, 9.90 million, 10.00 million,10.10 million, 10.20 million, 10.30 million, 10.40 million, 10.50 million, 10.60 million, 10.70 million, 10.80 million, 10.90 million, 11.00 million, 11.10 million, 11.20 million, 11.30 million, 11.40 million, 11.50 million, 11.60 million, 11.70 million, 11.80 million, 11.90 million, 12.00 million, 12.10 million, 12.20 million, 12.30 million, 12.40 million, 12.50 million, 12.60 million, 12.70 million, 12.80 million, 12.90 million, 13.00 million, 13.10 million, 13.20 million, 13.30 million, 13.40 million, 13.50 million, 13.60 million, 13.70 million, 13.80 million, 13.90 million, 14.00 million, 14.10 million, 14.20 million, 14.30 million, 14.40 million, 14.50 million, 14.60 million, 14.70 million, 14.80 million, 14.90 million, 15.00 million, 16.000 million, 17.000 million, 18.000 million, 19.000 million and 20.000 million of cells per kg of body weight.

Although these amounts of cells/kg body weight are described in relation to liver diseases, they are applicable as well in the treatment and/or prevention in the further diseases or conditions as described in the present application.

In some embodiments, the population of liver-derived cells is administered at a dose of about 0.25 to about 20 million cells per kg of body weight. In some embodiments, the population of liver-derived cells is administered at a dose of about 0.25 to about 15 million cells per kg of body weight. In some embodiments, the population of liver-derived cells is administered at a dose of about 0.25 to about 10 million cells per kg of body weight. In some embodiments, the population of liver-derived cells is administered at a dose of about 0.25 to about 5 million cells per kg of body weight. In some embodiments, the population of liver-derived cells is administered at a dose of about 0.25 to about 2.5 million cells per kg of body weight. In some embodiments, the population of liver-derived cells is administered at a dose of about 0.25 to about 1.5 million cells per kg of body weight. In some embodiments, the population of liver-derived cells is administered at a dose of about 0.5 to about 20 million cells per kg of body weight. In some embodiments, the population of liver-derived cells is administered at a dose of about 0.5 to about 15 million cells per kg of body weight. In some embodiments, the population of liver-derived cells is administered at a dose of about 0.5 to about 10 million cells per kg of body weight. In some embodiments, the population of liver-derived cells is administered at a dose of about 0.5 to about 5 million cells per kg of body weight. In some embodiments, the population of liver-derived cells is administered at a dose of about 0.5 to about 2.5 million cells per kg of body weight. In some embodiments, the population of liver-derived cells is administered at a dose of about 0.5 to about 1.5 million cells per kg of body weight. In some embodiments, the population of liver-derived cells is administered at a dose of about 1 to about 20 million cells per kg of body weight. In some embodiments, the population of liver-derived cells is administered at a dose of about 1 to about 15 million cells per kg of body weight. In some embodiments, the population of liver-derived cells is administered at a dose of about 1 to about 10 million cells per kg of body weight. In some embodiments, the population of liver-derived cells is administered at a dose of about 1 to about 5 million cells per kg of body weight. In some embodiments, the population of liver-derived cells is administered at a dose of about 1 to about 2.5 million cells per kg of body weight. In some embodiments, the population of liver-derived cells is administered at a dose of about 1 to about 1.5 million cells per kg of body weight. In some embodiments, the population of liver-derived cells is administered at a dose of about 1.25 million cells per kg of body weight.

In an aspect, the invention relates to the cell population as described above in any one of the embodiments, or the pharmaceutical composition as described above in any one of the embodiments, for use in treating a condition that may benefit from their engraftment in human tissue.

Alternatively, or in addition thereto, the effects of the cell population as described above in any one of the embodiments, or the pharmaceutical composition as described above in any one of the embodiments can also be used in treating a condition that may benefit from paracrine signaling. Said signaling involves the release of bioactive molecules by cells to influence nearby cells. These signaling molecules can have various effects, including immunomodulation (modulating the immune system), immunosuppression (suppressing immune responses), actions against bacteria, and fibrosis (excessive tissue scarring).

In an embodiment, the liver cell population or the pharmaceutical composition for use as described above in any one of the embodiments, is administered to a subject. In a further preferred embodiment, the metabolic defect of said administered cells is absent in said subject.

In an embodiment, the liver cell population or the pharmaceutical composition for use as described above in any one of the embodiments is a suspension of isolated cells that is suitable for intrahepatic, intrasplenic, intraportal, or intravenous administration.

The form of the cell population or pharmaceutical composition may depend on the chosen administration method, can be a suspension of cells or other systems allowing the engraftment and functionality of cells. In particular, the cells can be administered via injection (encompassing also catheter administration) or implantation, e.g. localised injection, systemic injection, intrasplenic injection, intraarticular injection, intraperitoneal injection, intraportal injection, injection to liver pulp, e.g., beneath the liver capsule, parenteral administration, or intrauterine injection into an embryo or foetus. When systemically and not locally injected, the cells may have an effect in a distant location either because such cells move in the bloodstream and engraft in distant locations (such internal organs or joints), or the proteins secreted by the cells reach specific cell types, thanks to bloodstream.

When administering a therapeutic composition comprising the cells, it may generally be formulated in a unit dosage. In any case, it may be desirable to include agents and/or adapt known methods for administering cells to patients that ensure viability of the cells, for example by incorporating the cells into a biopolymer or synthetic polymer. Examples of suitable biopolymers include, but are not limited to, fibronectin, fibrin, fibrinogen, thrombin, collagen, laminins, adhesion molecules, proteoglycans, hyaluronans, glycosaminoglycan chains, chitosan, alginate, natural or synthetically modified peptides that are derived from such proteins, and synthetic, biodegradable and biocompatible polymers. These compositions may be produced with or without cytokines, growth factors, and administered as a suspension or as a three-dimensional gel with the cells embedded there within.

As the method relates to the production of liver-derived cells from livers or parts thereof with metabolic defects, the cells may be produced and isolated from tissue which is not patient's own, to not re-introduce the same defects. Where administration of such cells to a patient is contemplated, it may be preferable that the liver tissue subjected to the method of the present invention to obtain the cells is selected such as to maximize, at least within achievable limits, the tissue compatibility between the patient and the administered cells, thereby reducing the chance of rejection of the administered cells by patient's immune system (e.g., graft vs. host rejection).

An issue concerning the therapeutic use of cells is the quantity necessary to achieve an optimal effect. Doses for administration may be variable, may include an initial administration followed by subsequent administrations; and can be ascertained by the skilled artisan by applying the teaching of the present disclosure. Typically, the administered dose or doses will provide for a therapeutically effective amount of the cells and it may require optimization of the amount of administered cells. Thus, the quantity of cells to be administered will vary for the subject being treated (e.g. between 10² to 10¹⁰ cells per each treatment in a cycle or for the entire cycle of treatment). However, the precise determination of a therapeutically effective dose may be based on factors individual to each patient, including their size, age, size tissue damage, and amount of time since the damage occurred.

Preferably, compositions comprising the liver-derived cells should contain a substantially homogeneous cell population as defined above and the amount of cells within each dose can be consequently adjusted.

The distribution, differentiation, and/or proliferation of the cells after their administration or implantation can be determined (as well as their activity after/before the administration of a different therapeutic agent) can be tested in human subject or in animal models (preferably a rodent).

In an aspect, the invention relates to a method of obtaining a population of isolated liver-derived cells, suited for use as a personalized medicine in a patient suffering from a liver disease, or condition and/or a disease or condition caused by increased vascular permeability and/or a disease related to cellular senescence, comprising isolating liver-derived cells from a donor liver or part thereof, with metabolic defects, wherein said metabolic defects of said donor liver are absent in said patient, and administering said cells to said patient.

In an aspect, the invention relates to a method of obtaining a population of isolated liver-derived cells, suited for use in a patient suffering from a liver disease, or condition and/or a disease or condition caused by increased vascular permeability and/or a disease related to cellular senescence, comprising isolating liver-derived cells from a donor liver or part thereof, with metabolic defects, preferably wherein said metabolic defects from said donor liver are absent in said subject.

In an aspect, the invention relates to a method of obtaining a population of isolated liver-derived cells, suited to be administered to a subject, said method comprising screening the subject for the presence of one or more metabolic liver defects, and isolating liver-derived cells from a donor liver or part thereof having metabolic defects.

In an aspect, the invention relates to a method of obtaining a population of isolated liver-derived cells, suited to be administered to a subject, wherein said liver-derived cells are chosen from the list of liver progenitor cells such as adult liver progenitor cells, hepatocyte progenitors, cholangiocyte progenitors, stellate cell progenitors, liver endothelial cell progenitors, and/or Kupffer cell progenitors.

In addition, cells and cell populations obtained by the present method may be administered or used in combination with another product (such as a drug, therapeutic agent, another cell type, or other biological material). This applies to any of the administrations and therapeutic uses described herein. In particular, the other therapeutic product may be administered substantially at the same time with the cells obtained by the present method (within the same pharmaceutical composition or in distinct pharmaceutical composition) or at different times (in distinct pharmaceutical compositions and in any order or frequency). Whether the other therapeutic product is administered separately or not from the cells obtained by the present method, the resulting effects may be synergic, that is the effects are superior to the additional ones that are expected, the negative effects of one of such components are mitigated or disappear, and/or the positive effects of one of such components are obtained by administering it at a lower amount or less frequently.

The cells obtained by the present method may be administered (being or not previously co-cultured in in vitro conditions) in combination with another cell type (e.g., primary human hepatocytes, ADHLSC Cells, or another human cell type or population being a primary, stem, mesenchymal, and/or progenitor cell such as the ones described in WO2006126219 and other progenitor or stem cells of hepatic origin) or its corresponding conditioned cell culture media, in form of a cell culture supernatant. The combination of the cells with another cell type may improve the therapeutic efficacy, engraftment, homing, repopulation, proliferation, and/or stability of one and/or the other cell type within the human body. The cells obtained by the present method may be administered as part of a formulation also comprising such another cell type, or may be administered separately, but in combination with, that other cell type, such as sequentially or simultaneously (in any order).

The combination of cells obtained by the present method with the conditioned cell culture medium of another cell type may provide cells with improved therapeutic efficacy, engraftment, proliferation, and/or stability within the human body, together or not with further useful properties related to the composition of the conditioned cell culture medium of the other cell type.

The invention will now be illustrated by means of the following examples, which do not limit the scope of the invention in any way.

### EXAMPLES

Although Example 1 relates to the preparation of a liver cell suspension from a liver of a donor suffering from MSUD, livers with any of the above listed metabolic defects could have been used.

### Example 1: Preparation of a primary liver cell suspension from a liver with metabolic defect

Human liver cells were obtained from the whole explanted liver of a living eligible donor suffering from Maple Syrup Urine Disease (MSUD), a genetic disorder that affects the metabolism of branched-chain amino acids, and who has received a normal liver by transplantation. Liver donors are screened and tested in compliance with the applicable laws relating to the minimization of the risks of transmitting a communicable disease, for example they must have been tested negative for HIV, HBV, HCV and Syphilis.

The liver cells were isolated using the technique of two-step collagenase perfusion at 37°C thanks to cannulae inserted into the accessible vasculature and connected with the perfusion pump(s). The major steps in the production of primary liver cell suspensions comprise the loosening of desmosomes, the liver tissue digestion, the liver disruption, the filtration of the recovered cell suspension, the liver cell separation by centrifugation and the cryopreservation of the final suspension of primary liver cells.

The liver tissue was perfused with a calcium complexation medium, containing a Ca²⁺ chelating agent as well as antibiotics, in order to remove the residual blood and to loosen cell junctions (desmosomes) by complexing Ca²⁺. Digestion medium containing collagenases +/- protease was then infused into the liver, rapidly breaking down the collagen matrix. The liver tissue was further mechanically disrupted and lacerated. The cells were released from the digested tissue by gentle shaking, and then filtered and rinsed through metal sieves in order to remove undigested liver pieces. The liver cell suspension was then washed 2 times by centrifugation in a cold wash medium and then concentrated before freezing and storage in bags. Optionally, freshly prepared primary liver cell suspension may be used and directly processed for manufacturing of the liver progenitor cell population after quality control assessment.

### Example 2: Preparation of human adult liver-derived progenitor cells (HALPC)

Liver cell preparations obtained in example 1 are cultured under stringent culture conditions as previously described. Briefly, liver cell preparations are re-suspended in Williams' E medium supplemented with 10% FBS, 10 mg/ml INS, 1 mM DEX. The primary cells are cultured on Corning^{®} CellBIND^{®} flasks and cultured at 37°C in a fully humidified atmosphere containing 5% CO₂. After 24 hours, medium is changed in order to eliminate the non-adherent cells and thereafter renewed twice a week, whereas the culture is microscopically followed every day until P1. Culture medium is switched after 12- 16 days to high glucose DMEM supplemented with 9% FBS. A cell type with mesenchymal-like morphology emerges and proliferates. When reaching 70-95% confluence, cells are trypsinized with recombinant trypsin and 1 mM EDTA and re- plated at a density of 1-10 × 10³ cells/cm². At each passage, cells were trypsinized at 90% confluency.

Functionality such as immunomodulatory activity and secretome analysis and cell viability were tested and found to be generally comparable between the liver progenitor cells obtained from a normal liver. Identity and purity testing of the HALPCs produced from liver with metabolic defect, was also found to be comparable, and met acceptance criteria established for the HALPCs.

Cell identity and impurities analyses can be assessed to confirm that they express, inter alia, the following markers: CD90, CD73, Vimentin, and ASMA, and are negative, or exhibit very low expression, with respect to the following markers: CD133, CD45, CK19 and CD31. The residual process impurity (BSA) and the potency via PGE2 secretion are determined through specific ELISAs. Additional tests to characterize the drug product can be used to evaluate for example cell density and cell morphology, or to further investigate the potency of the cells, or to verify their cytogenetic stability via karyotyping.

### Example 3: Administration of HALPCs to patients with ACLF or at risk of developing ACLF

"MELD" is an acronym for the "Model for End-stage Liver Disease" scoring system which is used for assessing severity of end-stage liver disease. MELD scores are used in the art to predict mortality, and also to stratify patients (over 12 years old) in respect of the need for a liver transplant. The scoring is based on values of a patient's serum creatinine, bilirubin, INR (international normalized ratio of prothrombin time) and is determined according to the following formula: 9.57 × log e (creatinine mg/dL) + 3.78 × log e (bilirubin mg/dL) + 11.2 × log e (INR) + 6.43. The resulting score is usually rounded to the nearest integer.

The "Child-Pugh score", also referred to as "Child-Turcotte-Pugh score" or "Child Criteria", is used to assess the prognosis of chronic liver disease.

HALPC as prepared in example 2 could be administered to patients affected with ACLF or at risk of developing ACLF, as described in Nevens et al., 2021. In this clinical study, fourteen patients with confirmed acute-on-chronic liver failure (ACLF) and eight patients with acute decompensation (AD), with risk of developing ACLF, were treated with HALPCs, using the dosing regimen as shown below. The cells were counted using the manual method described above.

The MELD score of the patients prior to treatment ranged from 18 to 35, with an average of about 27. The total bilirubin serum concentration of each patient was higher than 6 mg/dL (≥100 umol/L); between the patients, it ranged from about 7 to about 43mg/dL with an average of about 22 mg/dL. All patients received standard medical treatment (SMT) as required by their clinical status, but no concomitant anticoagulant therapy.

In total, 22 patients were subjected to the treatment as follows:
Six patients received one infusion of about 0.6 million cells/kg. Three further patients received two infusions of about 0.6 million cells/kg at an interval of about 7 days..
Four patients received one infusion with about 1.2 million cells/kg. Nine patients received two infusions of about 1.2 million cells/kg at an interval of about 7 days. In all cases, the infused cells are comprised in compositions that contain only pharmacologically insignificant amounts of heparin, i.e. not more than 10 I.U. per kg body weight. None of the patients received anti-coagulant co-medication.

The MELD (particularly relevant for transplant prioritization) and the Child-Pugh (particularly relevant for evaluating the mortality risk) scores in the patients were assessed. Further, hematology and serum biochemistry markers such as bilirubin, creatinine and sodium levels were assessed.

### Example 4: Efficacy and safety of HALPCs in patients with ACLF

A randomized, placebo-controlled, double blind, multi-centre study is conducted to evaluate the efficacy and safety of HALPCs in patients with Acute on Chronic Liver Failure (ACLF). Patients recently diagnosed with ACLF grade 1 or 2 are proposed to undergo the screening procedures to participate in the study. ACLF grading are based on the CLIF Organ Failure (CLIF-OF) score. Patients are at least 18 years old and have a bilirubin value of at least 5 mg/dL. Moreover, the patients enrolled in the study have a MELD score of not higher than 35 and are free of an underlying cirrhosis due to biliary disease or autoimmune hepatitis.

The patients participating in the study are then randomised and allocated either to receive the best standard of care treatment plus a placebo, or the best standard of care plus two infusions of HALPCs at an interval of about 1 week, each infusion containing an HALPC dose of about 1 million cells per kg body weight. The HALPC infusions are administered as compositions that do not comprise any pharmacologically relevant amounts of anticoagulants, in particular not more than 10 I.U./kg heparin per infusion.

An evaluation period of 3 months post-infusion will follow the treatment regimen in which the safety and efficacy data will be obtained from the patients.

It is expected that this further study will confirm the efficacy of HALPCs in patients with ACLF using a dosage regimen as discussed above. More specifically, it will also demonstrate the efficacy of 2 infusions (i.v.) of HALPCs, each containing 1.0 million of cells/kg, administered at a 7-day interval, in terms of a positive effect on the overall survival proportion of the patients 90 days post-first infusion.

### Example 5: Administration of HALPCs to patients with NASH

The safety of HALPCs as prepared in example 2 in NASH patients could be evaluated according to method used for the HEP201 (PANASH) study, as described hereafter. HEP201 (PANASH) study protocol:
Adult patients with either fibrosis stage F3 or F4 NASH (SAF fibrosis score) received a single or 3 repeated weekly infusions of either 0.5 or 1.0 × 10⁶ cells of HepaStem (HALPCs)/kg body weight and were followed up for 6 months post infusion.

Twenty-three patients in total were included in the study, all compensated except for 2 early decompensated (total bilirubin > 2 mg/dL)): 11 patients had fibrosis stage F3, and 12 patients had fibrosis stage F4.

The patients (e.g. 23) were allocated to the following dose cohorts, each of which may, e.g., include some patients with fibrosis stage F3 and some patients with fibrosis stage F4:
Cohort 1: Single infusion of 0.5 × 10⁶ cells/kg, 6 patients: 3 F3 and 3 F4
Cohort 2: Single infusion of 1.0 × 10⁶ cells/kg, 6 patients: 3 F3 and 3 F4
Cohort 3: Three infusions of 0.5 × 10⁶ cells/kg at intervals of 7 days, 7 patients: 3 F3 and 4 F4
Cohort 4: Three infusions of 1.0 × 10⁶ cells/kg at intervals of 7 days, 4 patients: 2 F3 and 2 F4.

In a stepwise safety approach, cohort 1 received the specified single dose, and upon observing that the dose was safe, the escalation to the next dose cohort was done. Similarly, the escalation from dose cohort 2 to 3 and from cohort 3 to 4 was performed.

Up to 3 infusions at 1.0 × 10⁶ cells HepaStem/kg body weight were safe and well tolerated. Serum levels of ALT (alanine transaminase) and AST (aspartate aminotransferase), which were mildly elevated at baseline, tended to normalize by Month 6, particularly in F3 patients. Bilirubin, as well as triglyceride levels, gradually decreased over time, particularly in patients with higher values at baseline.

### Example 6: Administration of HALPCs to patients affected by Urea Cycle disorders or Crigler-Najjar Syndrome

HALPC as prepared in example 2 could be administered to patients affected by Urea Cycle disorders or Crigler-Najjar Syndrome, using the clinical protocol described in Smets and al., 2019.

In summary, this phase I/II prospective, open label, multicenter, randomized trial primarily evaluated the safety of HepaStem in pediatric patients with urea cycle disorders (UCDs) or Crigler-Najjar (CN) syndrome 6 months post-transplantation. The secondary objective included the assessment of safety up to 12 months post-infusion and of preliminary efficacy.

Fourteen patients with UCDs and 6 with CN syndrome were divided into 3 cohorts by body weight (BW) (cohort 1: BW> 20 kg, cohort 2: BW 10-20 kg, cohort 3: BW < 10 kg) and intraportally infused with 3 different doses of HepaStem (low: 12.5 × 10⁶ cells/kg, intermediate: 50 × 10⁶ cells/kg and high: 200 × 10⁶ cells/kg), with a maximum of 4 × 10⁹ total cells infused. Clinical status, portal vein hemodynamics, morphology of the liver, de novo detection of circulating anti-human leukocyte antigen antibodies, and clinically significant adverse events (AEs) and serious adverse events to infusion were evaluated by using an intent-to-treat analysis. The overall safety of HepaStem was confirmed.

### Example 7: In-vivo efficacy of liver-derived cells produced from liver with metabolic defect in ACLF disease using mouse models

According to the clinical trials conducted so far with HepaStem and targeting ACLF disease, ACLF mouse models are implemented to evaluate in vivo the potential of HALPCs and other liver-derived cells produced from liver with metabolic defect in such indication. Based on recent literature information (Engelmann et al., 2020; Wu et al., 2022), mice are treated 6 weeks with CCl4 and then injected with lipopolysaccharide, a bacterial endotoxin, or D-galactosamine, an important experimental hepatotoxin, which causes acute hepatitis. Both animal models are manageable, easy to implement and well characterized. The appropriate animal model will be selected for the evaluation of the cell products.

The aim of the in vivo study will be to evaluate the efficacy of liver-derived cells produced from liver with metabolic defect in the selected ACLF mouse model. Several specific disease-relevant parameters will be followed including animal mortality, cell death, levels of transaminases and bilirubin, macrophage infiltration, fibrosis, parenchymal regeneration as well as immune pro- and anti-inflammatory cell populations. Such inflammation markers will be analyzed both at the tissular and peripheral level.

## Claims

1. A method for obtaining a population of isolated liver-derived cells suitable for therapeutic use, wherein said liver-derived cells are obtained from isolated adult livers with metabolic defects or a part thereof, preferably obtained from one isolated adult liver with one metabolic defect, or a part thereof.

2. The method according to claim 1, wherein said adult livers with metabolic defects or parts thereof are adult livers or parts thereof displaying impaired metabolic functions linked to genetic mutations and/or enzymatic deficiencies, preferably wherein said genetic mutations or enzymatic deficiencies result in a metabolic liver disease or disorder, wherein said liver disease or disorder is preferably chosen from the group of familial amyloidotic polyneuropathy (FAP), maple syrup urine disease (MSUD), acute intermittent porphyria, hereditary fibrinogen A o-chain amyloidosis, propionic acidemia, hyperhomocysteinemia, methylmalonic acidemia, familial hypercholesterolemia, primary hyperoxaluria, hemophilia A, urea cycle disorders, and Crigler Najjar syndrome.

3. The method according to claim 1 or 2, wherein said adult liver or part thereof is obtained from a donor, preferably a human donor, wherein said liver is isolated from said donor, or wherein said part of said liver is obtained from said donor via a biopsy, wherein the method preferably comprises a step of screening said donor for the presence of metabolic liver diseases or disorders, and/or wherein the method preferably comprises a step of screening said adult liver or part thereof for the presence of metabolic defects.

4. The method according to any one of the previous claims, wherein the liver with metabolic defects or a part thereof shows an unaltered structure compared to such liver or part thereof without said metabolic defects.

5. The method according to any one of the previous claims, wherein said liver-derived cells are chosen from liver progenitor cells such as adult liver progenitor cells, hepatocyte progenitors, cholangiocyte progenitors, stellate cell progenitors, liver endothelial cell progenitors, and/or Kupffer cell progenitors.

6. A suspension of primary liver cells obtained from isolated adult livers with metabolic defects or a part thereof, preferably obtained from one isolated adult liver with one metabolic defect, or a part thereof, wherein said primary liver cells preferably have a metabolic defect.

7. A liver cell population obtained from isolated adult livers with metabolic defects or a part thereof, comprising liver-derived cells obtained by a method of any one of the claims 1 to 5, or obtained by culturing and expanding the suspension of primary liver cells according to claim 6 and subsequently harvesting said expanded cell population.

8. The liver cell population according to claim 7 or the suspension of primary liver cells according to claim 6, wherein the cells have metabolic defects, preferably one metabolic defect, said metabolic defects are linked to one or more genetic mutations and/or enzymatic deficiencies resulting in a metabolic liver disease or disorder, said liver disease or disorder is preferably chosen from the group of familial amyloidotic polyneuropathy (FAP), maple syrup urine disease (MSUD), acute intermittent porphyria, hereditary fibrinogen A o-chain amyloidosis, propionic acidemia, hyperhomocysteinemia, methylmalonic acidemia, familial hypercholesterolemia, primary hyperoxaluria, hemophilia A, urea cycle disorders, and Crigler Najjar syndrome.

9. A pharmaceutical composition comprising a therapeutically effective amount of the liver cell population according to any one of the claim 7 or 8.

10. The liver cell population of any one of claim 7 or 8, or the pharmaceutical composition of claim 9 for use as a medicament.

11. The liver cell population of any one of claim 7 or 8, or the pharmaceutical composition of claim 9 for use in treating a liver disease or condition in a subject, preferably wherein the liver disease or condition is selected from the group of phenylketonuria and other aminoacidopathies, hemophilia and other clotting factor deficiencies, familial hypercholesterolemia and other lipid metabolism disorders, urea cycle disorders, glycogenosis, galactosemia, fructosemia, tyrosinemia, protein and carbohydrate metabolism deficiencies, organic aciduria, mitochondrial diseases, peroxysomal and lysosomal disorders, protein synthesis abnormalities, defects of liver cell transporters, defects of glycosylation, cirrhosis, inborn errors of metabolism, acute liver failure, acute liver infections, acute chemical toxicity, chronic liver failure, fulminant liver failure, cholangitis, biliary cirrhosis, Alagille syndrome, alpha-1-antitrypsin deficiency, autoimmune hepatitis, biliary atresia, cancer of the liver including hepatocellular carcinoma (HCC), cystic disease of the liver, gallstones, Gilbert's syndrome, hemochromatosis, hepatitis A, hepatitis B, hepatitis C, and other hepatitis viral infections, porphyria, primary sclerosing cholangitis, Reye's syndrome, sarcoidosis, type 1 glycogen storage disease, Wilson's disease, an inherited Blood Coagulation Disorder, progressive familial intrahepatic cholestasis type 1 / 2 / 3, defect of liver cell transporters, fatty liver, fibrotic liver disease, Acute-on- chronic liver failure (ACLF) and non-alcoholic fatty liver disease (NAFLD), nonalcoholic steatohepatitis (NASH), or liver degenerative disease.

12. The liver cell population of any of the claim 7 or 8, or the pharmaceutical composition of claim 9, for use in modulating or influencing impaired vascular permeability in (cells of) a subject; and/or for use in the treatment of diseases and/or conditions caused by increased vascular permeability, preferably wherein said diseases and/or conditions caused by increased vascular permeability are chosen from the group of heart, pulmonary and ischemic diseases, diabetes and ocular diseases, cancer (solid tumors), Clarkson's disease and sepsis in a subject, preferably triggered by an infection and/or is sepsis or a sepsis-induced disease such as sepsis-induced myocardial edema, sepsis-induced acute kidney injury, lung sepsis, or Clarkson's disease.

13. The liver cell population of any of the claim 7 or 8, or the pharmaceutical composition of claim 9, for use in the treatment and/or prevention of one of more diseases related to cellular senescence selected from the group comprising or consisting of hepatic fibrosis, pre-cirrhotic conditions, cirrhosis including liver and biliary cirrhosis, biliary atresia, Alagille syndrome, progressive familial intrahepatic cholestasis, primary biliary cholangitis, primary sclerosing cholangitis, chronic hepatitis, chronic hepatitis B virus (HBV) infection, chronic hepatitis C virus (HCV) infection, cholestasis, osteoporosis, osteoarthritis, atherosclerosis, cardiac hypertrophy, cardiac fibrosis, cardiomyopathy, thrombosis, cataracts, glaucoma, macular degeneration, chronic obstructive pulmonary disease (COPD), pulmonary fibrosis, renal dysfunction, pancreatic fibrosis, type 2 diabetes, Alzheimer disease, Huntington's disease, Parkinson's disease, dementia, lipodystrophy, sarcopenia, age-related cachexia, skin aging, hepatocellular carcinoma, liver cancer, lobular carcinoma, bile duct cancer, melanoma, lung cancer, and islet cell tumor.

14. The liver cell population or pharmaceutical composition for use of claim 13, wherein said liver cell population or pharmaceutical composition is administered to a subject, and wherein the metabolic defect of said administered cells is absent in said subject.

15. A method of obtaining a population of isolated liver-derived cells, suited for use in a patient suffering from a liver disease or condition and/or a disease or condition caused by increased vascular permeability and/or a disease related to cellular senescence, comprising isolating liver-derived cells from a donor liver or part thereof with metabolic defects, preferably wherein said metabolic defects from said donor liver are absent in said subject.
